# EUROPEAN PATENT APPLICATION

(11) **EP 2 441 768 A1**
(43) Date of publication of application: **18.04.2012**
(21) Application number: 11196260.1
(22) Date of filing: 13.11.2007
(51) Int. Cl.: C07D 495/04, A61K 31/519, A61P 29/00, A61P 35/00

(54) **Thienopyrimidinones for treatment of inflammatory disorders and cancers**

(30) Priority: 13.11.2006 US 858850 P
(62) Divisional of application: 07864345.9
(71) Applicant: Eli Lilly & Co., Indianapolis, IN 46285 (US)
(72) Inventor: White, Stephen, L., Boyds, MD 20841 (US); Kesicki, Edward, A., Bothell, WA 98021 (US); Thorsett, Eugene, Half Moon Bay, CA 94019 (US); Ruan, Fuqiang, Bellevue, WA 98006 (US); Farouz, Francine, La Jolla, CA 92037 (US)
(74) Representative: Lord, Hilton David

(57) **Abstract**

The current invention provides compounds of formula (I); wherein one of Q¹, Q² and Q³ is S, and the other of two of Q¹, Q² and Q³ are -CR¹-, which are inhibitors of P13K-delta. These compounds are useful for treatment of conditions mediated by P13K-delta, such as hematopoietic cancers, immune disorders, and bone resorption disorders. The invention further provides pharmaceutical compositions comprising a compound of formula (I) and methods of using these compounds and compositions to treat conditions mediated by P13K-delta.

## Description

### FIELD OF THE INVENTION

The invention relates generally to novel compounds that are selective inhibitors of PI3Kδ, and are useful for treating and/or preventing conditions associated with aberrant proliferation of hematopoietic cells and various conditions characterized by inflammation. More particularly, the invention relates to compositions and methods for treating and/or preventing certain cancers, especially hematologic cancers such as leukemia, as well as various inflammatory and immune disorders, including rheumatoid arthritis and asthma.

### BACKGROUND OF THE INVENTION

Cell signaling via 3'-phosphorylated phosphoinositides has been implicated in a variety of cellular processes, *e.g*., malignant transformation, growth factor signaling, inflammation, and immunity (see Rameh, et al., J. Biol. Chem. (1999) 274:8347-8350 for a review). The enzymes responsible for generating these phosphorylated signaling products, phosphatidylinositol 3-kinase (PI 3-kinase; PI3K), were originally identified as an activity associated with viral oncoproteins and growth factor receptor tyrosine kinases that phosphorylates phosphatidylinositol (PI) and its phosphorylated derivatives at the 3'-hydroxyl of the inositol ring (Panayotou, et al., Trends Cell Biol. (1992) 2:358-360).

Presently, three classes of PI 3-kinase (PI3K) enzymes are distinguished, based on their substrate specificities. Class I PI3K's can phosphorylate phosphatidylinositol (PI), phosphatidylinositol-4-phosphate, and phosphatidylinositol-4,5-diphosphate (PIP2) to produce phosphatidylinositol-3-phosphate (PIP), phosphatidylinositol-3,4-diphosphate, and phosphatidylinositol-3,4,5-triphosphate, respectively. Class II PI3K's phosphorylate PI and phosphatidylinositol-4-phosphate, whereas Class III PI3K's can only phosphorylate PI.

The initial purification and molecular cloning of PI 3-kinase revealed that it was a heterodimer consisting of p85 and p110 subunits (Otsu, et al., Cell (1991) 65:91-104; Hiles, et al., Cell (1992) 70:419-429). Since then, four distinct Class I PI3K's have been identified, designated PI3Kα, β, γ, and δ, each consisting of a distinct 110 kDa catalytic subunit and a regulatory subunit. More specifically, three of the catalytic subunits, *i.e*., p110α, p110β, and p110δ, each interact with the same regulatory subunit, p85; whereas p110γ interacts with a distinct regulatory subunit, p101. As described below, the patterns of expression of each of these PI3K's in human cells and tissues are also distinct.

Identification of the p110δ isoform of PI 3-kinase is described in Chantry, et al., J Biol Chem (1997) 272:19236-19241. It was observed that the human p110δ isoform is expressed in a tissue-restricted fashion. It is expressed at high levels in lymphocytes and lymphoid tissues, suggesting that the protein might play a role in PI 3-kinase-mediated signaling in the immune system. Details concerning the p110δ isoform can be found in U.S. Patent Nos. 5,858,753; 5,822,910; and 5,985,589. See also, Vanhaesebroeck, et al., Proc Natl. Acad. Sci. USA (1997) 94:4330-4335, and published PCT application WO 97/46688.

The delta (δ) isoform of PI3K is primarily expressed in cells of hematopoietic origin; consequently, selective inhibitors of this isoform are expected to primarily affect hematopoietic cells. Such cells are central to the various functions of the blood, and consequently they are critically involved in disorders characterized by inflammation and immune responses as well as disorders of the blood and cardiovascular system. Selective inhibitors are therefore expected to be useful for treatment of such disorders with minimal effect on other systems and processes mediated by other isoforms of PI3K. Indeed, selective inhibitors of PI3Kδ have been demonstrated in animal models to reduce allergic inflammation of airways and hyperresponsiveness in an animal model for asthma. Lee, K. S., et al., FASEB Journal (2006) 20:455-465. Selective PI3Kδ inhibitors have also been shown to inhibit proliferation of aberrant leukocytes in acute myeloid leukemia (AML), without affecting proliferation of normal hematopoietic cells. Sujobert, P., et al., Blood (2005) 106:1063-1066. The selectivity for the delta isoform over other isoforms, especially the alpha, beta and gamma isoforms, is thus an important aspect of providing a compound that is useful to treat disorders associated with excessive accumulation, activity or production of hematopoietic cells, without preventing the function and/or proliferation of normal hematopoietic cells.

Selective inhibitors of PI3Kδ are disclosed, for example, in U.S. Patent Nos. 6,518,277; 6,667,300; 6,949,535; and 6,800,620, and in published U.S. Patent Application US 2006/0106038 and PCT application WO 2005/113554. However, a need remains for additional therapeutic agents useful to treat proliferative disorders, such as cancer, and excessive or destructive immune reactions, such as asthma, rheumatoid arthritis, multiple sclerosis, and lupus. The present invention provides novel compounds that are potent inhibitors of PI3Kδ, and are highly selective for the delta isoform and much less active against other isoforms of PI3K. These compounds are useful for the treatment of disorders associated with excessive activity, accumulation or production of hematopoietic cells, especially lymphocytes and leukocytes, including lymphomas, leukemias, and excessive immune response disorders.

### SUMMARY OF THE INVENTION

The invention provides novel compounds that selectively or specifically inhibit PI3Kδ activity, and are thus useful both therapeutically and prophylactically, for the treatment of disorders wherein a subject experiences excessive activity, accumulation, or production of cells that express PI3Kδ. The invention includes compounds, pharmaceutical compositions, and methods of administering the compounds and compositions for the treatment of hematologic malignancies, inflammation, autoimmune disorders, allergic reactions, and cardiovascular disease. This method can be employed in treating humans or animals that are afflicted by or may be subject to any condition whose symptoms or pathology is mediated by PI3Kδ expression or activity, or is characterized by excessive production, activity or accumulation of cells that express PI3Kδ. The compositions and methods of the invention are particularly effective for the treatment of hematologic cancers such as acute myelogenous leukemia (AML), acute lymphocytic leukemia (ALL), chronic myelogenous leukemia (CML), mastocytosis, chronic lymphocytic leukemia (CLL), myelodysplastic syndrome (MDS), and multiple myeloma (MM); autoimmune disorders such as rheumatoid arthritis, multiple sclerosis, scleroderma, Sjögren's syndrome, multiple sclerosis, myasthenia gravis, Guillain-Barré syndrome, Hashimoto's thyroiditis, Graves' disease, inflammatory bowel disease (IBD: Crohn's disease, ulcerative colitis), vasculitis, hemolytic anemia, thrombocytopenia, psoriasis, type I (insulin dependent) diabetes, and systemic lupus erythematosus (SLE); and excessive immune system reactions, such as asthma and allergic rhinitis.

The invention provides compounds of formula (1) and pharmaceutical compositions comprising compounds of formula (1), as well as methods of using these compounds and compositions. The compounds of the invention have this formula: 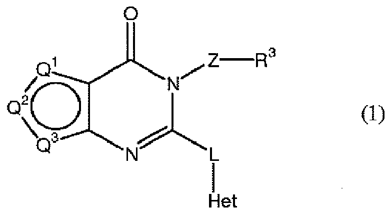 wherein:
one of Q¹, Q² and Q³ is S, and the other of two of Q¹, Q² and Q³ are -CR¹-;
   wherein each R¹ is independently H, halo, OR, NR₂, NROR, NRNR₂, SR, SOR, SO₂R, SO₂NR₂, NRSO₂R, NRCONR₂, NRCOOR, NRCOR, CF₃, CN, COOR, CONR₂, OOCR, COR, or NO₂,
   or R¹ can be an optionally substituted member selected from the group consisting of C1-C8 alkyl, C2-C8 heteroalkyl, C2-C8 alkenyl, C2-C8 heteroalkenyl, C2-C8 alkynyl, C2-C8 heteroalkynyl, C1-C8 acyl, C2-C8 heteroacyl, C6-C10 aryl, C5-C12 heteroaryl, C7-C12 arylalkyl, and C6-C12 heteroarylalkyl groups,
   wherein each R is independently H or C1-C8 alkyl, C2-C8 heteroalkyl, C2-C8 alkenyl, C2-C8 heteroalkenyl, C2-C8 alkynyl, C2-C8 heteroalkynyl, C1-C8 acyl, C2-C8 heteroacyl, C6-C10 aryl, C5-C10 heteroaryl, C7-C12 arylalkyl, or C6-C12 heteroarylalkyl,
   and wherein two R on the same atom or on adjacent atoms can be linked to form a 3-8 membered ring, optionally containing one or two N, O or S as ring members;
   and wherein each R group other than H, and each ring formed by linking two R groups together, is optionally substituted;
Z is a bond, or is O, NR², C1-C6 alkylene or C 1-C6 heteroalkylene, each of which is optionally substituted with up to two C1-C6 alkyl or C2-C6 heteroalkyl groups, where two of said alkyl or heteroalkyl groups can optionally cyclize to form a 3-7 membered ring containing up to two heteroatoms selected from O, N and S as ring members;
R³ is aryl, heteroaryl, cycloalkyl, or heterocycloalkyl, each of which is optionally substituted with up to three R¹,
   or R³ can be H if Z is not a bond;
L is selected from the group consisting of -C(R²)₂-, -C(R²)₂-C(R²)₂-, -C(R²)₂-NR²- , and -C(R²)₂-S(O)ₙ-,
   wherein each R⁹ is independently H or an optionally substituted member selected from C1-C6 alkyl, C2-C6 heteroalkyl, C2-C6 alkenyl, and C2-C6 alkynyl, and n is 0-2;
   and two R², if present on L, can cyclize to form a 3-7 membered ring that may contain up to two heteroatoms selected from N, O and S as ring members;
Het is a monocyclic or bicyclic ring system wherein at least two ring atoms are N and wherein at least one ring is aromatic, and Het is optionally substituted with up to three substituents selected from R⁴, N(R⁴)₂, S(O)ₚR⁴, OR⁴, halo, CF₃, CN, NR⁴OR⁴, NR⁴N(R⁴)₂, SR⁴, SOR⁴, SO₂R⁴, SO₂N(R⁴)₂, NR⁴SO₂R⁴, NR⁴CON(R⁴)₂, NR⁴COOR⁴, NR⁴COR⁴, CN, COOR⁴, CON(R⁴)₂, OOCR⁴, COR⁴, or NO₂,
   wherein each R⁴ is independently H or an optionally substituted member selected from the group consisting of C1-C8 alkyl, C2-C8 heteroalkyl, C2-C8 alkenyl, C2-C8 heteroalkenyl, C2-C8 alkynyl, C2-C8 heteroalkynyl, C1-C8 acyl, C2-C8 heteroacyl, C6-C10 aryl, C5-C10 heteroaryl, C7-C12 arylalkyl, and C6-C12 heteroarylalkyl,
   and wherein two R⁴ on the same atom or on adjacent atoms can be linked to form a 3-8 membered ring, optionally containing one or two heteroatoms selected from N, O and S;
   wherein the optional substituents on each optionally substituted alkyl, heteroalkyl, alkenyl, heteroalkenyl, alkynyl, heteroalkynyl, acyl, heteroacyl, aryl, heteroaryl, arylalkyl and heteroarylalkyl, are selected from C1-C4 alkyl, halo, CF₃, CN, =O, =N-CN, =N-OR', =NR', OR', NR'₂, SR', SO₂R', SO₂NR'₂, NR'SO₂R', NR'CONR'₂, NR'COOR', NR'COR', CN, COOR', CONR'₂, OOCR', COR', and NO₂,
   wherein each R' is independently H, C1-C6 alkyl, C2-C6 heteroalkyl, C1-C6 acyl, C2-C6 heteroacyl, C6-C10 aryl, C5-C10 heteroaryl, C7-12 arylalkyl, or C6-12 heteroarylalkyl, each of which is optionally substituted with one or more groups selected from halo, C1-C4 alkyl, C1-C4 heteroalkyl, C1-C6 acyl, C1-C6 heteroacyl, hydroxy, amino, and =O;
   and wherein two R' on the same or adjacent atoms can be linked to form a 3-7 membered ring optionally containing up to three heteroatoms selected from N, O and S; and
   p is 0-2;
or a pharmaceutically acceptable salt thereof.

The invention provides methods for treating and/or preventing aberrant proliferation of hematopoietic cells that includes selectively inhibiting phosphoinositide 3-kinase delta (PI3Kδ) activity in hematopoietic cells by administering a compound of formula (1) or a pharmaceutical composition thereof. In one aspect, the method involves administering an amount of a PI3Kδ selective inhibitor of formula (1) that is effective to inhibit PI3Kδ activity of hematopoietic cells. In another aspect, the PI3Kδ selective inhibitor is administered in an amount effective to inhibit Akt phosphorylation in hematopoietic cells. In an additional aspect, the PI3Kδ selective inhibitor is administered in an amount effective to inhibit FOXO3a phosphorylation in hematopoietic cells. In a further aspect, the PI3Kδ selective inhibitor is administered in an amount effective to inhibit GAB 1 phosphorylation in hematopoietic cells. In a further aspect, the PI3Kδ selective inhibitor is administered in an amount effective to inhibit GAB2 phosphorylation in hematopoietic cells.

In one aspect, the methods are carried out *ex vivo,* by contacting a PI3Kδ with a compound of formula (1). In another aspect, the methods are carried out *in vivo,* by administration of a compound of formula (1) or a composition containing a compound of formula (1) to a subject diagnosed as having a condition associated with undesired proliferation or activity or accumulation of a hematopoietic cell type that expresses PI3Kδ. The methods may generally be used to treat any indication involving aberrant proliferation of lymphoid and/or myeloid progenitor cells. In one aspect, the indication can be acute lymphoblastic leukemia; acute myeloid leukemia; chronic lymphocytic leukemia; chronic myelogenous leukemia; hairy cell leukemia; polycythemia vera; chronic idiopathic myelofibrosis; essential thrombocythemia; refractory anemia; refractory anemia with ringed sideroblasts; refractory anemia with excess blasts; refractory anemia with excess blasts in transformation; Hodgkin's lymphoma; B-cell lymphoma; Burkitt's lymphoma; diffuse cell lymphoma; follicular lymphoma; immunoblastic large cell lymphoma; lymphoblastic lymphoma; mantle cell lymphoma; mycosis fungoides; post-transplantation lymphoproliferative disorder; small non-cleaved cell lymphoma; T-cell lymphoma; or, plasma cell neoplasms. The methods are particularly effective when the PI3K pathway is constitutively activated in the hematopoietic cells.

The methods may further include administering a mammalian target of rapamycin (mTOR) inhibitor. In one aspect of this embodiment, the mTOR inhibitor is selected from rapamycin, FK506, cyclosporine A (CsA), and everolimus.

In another embodiment, the invention provides methods for treating and/or preventing leukemia by selectively inhibiting phosphoinositide 3-kinase delta (PI3Kδ) activity in leukemic cells. In one aspect, the methods include administering an amount of a PI3Kδ selective inhibitor effective to inhibit PI3Kδ activity of leukemic cells. In another aspect, a PI3Kδ selective inhibitor is administered in an amount effective to inhibit Akt phosphorylation in leukemic cells. In a further aspect, a P13KS selective inhibitor is administered in an amount effective to inhibit FOXO3a phosphorylation in leukemic cells. In a further aspect, a PI3Kδ selective inhibitor is administered in an amount effective to inhibit GAB1 phosphorylation in leukemic cells. In a further aspect, a PI3Kδ selective inhibitor is administered in an amount effective to inhibit GAB2 phosphorylation in leukemic cells.

PI 3-kinase also appears to be involved in a number of aspects of leukocyte activation. A p85-associated PI 3-kinase activity has been shown to physically associate with the cytoplasmic domain of CD28, which is an important costimulatory molecule for the activation of T-cells in response to antigen (Pages, et al., Nature (1994) 369:327-329; Rudd, Immunity (1996) 4:527-534). Activation of T cells through CD28 lowers the threshold for activation by antigen and increases the magnitude and duration of the proliferative response. These effects are linked to increases in the transcription of a number of genes including interleukin-2 (IL2), an important T cell growth factor (Fraser, et. al., Science (1991) 251:313-316). Mutation of CD28 such that it can no longer interact with PI 3-kinase leads to a failure to initiate IL2 production, suggesting a critical role for PI 3-kinase in T cell activation.

The compounds of the invention are also useful for disrupting leukocyte function. Thus the invention also provides a method for disrupting leukocyte function by contacting leukocytes with a compound that selectively inhibits phosphatidylinositol 3-kinase delta (PI3Kδ) activity in the leukocytes. According to the method, the leukocytes can be neutrophils, B lymphocytes, T lymphocytes, or basophils.

For example, in cases in which the leukocytes are neutrophils, the method can include disrupting at least one neutrophil function such as stimulated superoxide release, stimulated exocytosis, or chemotactic migration. Preferably, the method does not substantially disrupt bacterial phagocytosis or bacterial killing by the neutrophils. In cases wherein the leukocytes are B lymphocytes, the method can include disrupting proliferation of the B lymphocytes or antibody production by the B lymphocytes. In cases wherein the leukocytes are T lymphocytes, the method can involve disrupting proliferation of the T lymphocytes. In cases wherein the leukocytes are basophils, the method can include disrupting histamine release by the basophils.

PI3Kδ is also involved in the accumulation of neutrophils in inflamed tissues, and a selective inhibitor of PI3Kδ blocks that effect in an animal model using lipopolysaccharide to induce inflammation in the trachea. Puri, K. L., Curr. Enz. Inhib. (2006) 2:147-161. Furthermore, a selective PI3Kδ inhibitor also inhibited allergic responses in an animal model. *Id*. In addition, the selective inhibitor demonstrated anti-hypertensive activity in an animal model. *Id*. Thus selective inhibitors of PI3Kδ are known to be useful to treat a variety of medical conditions, and the selective PI3Kδ inhibitors of the invention are useful to treat inflammation by reducing accumulation of neutrophils in affected tissues, and to reduce the severity of allergic reactions, as well as to treat hypertension.

### DETAILED DESCRIPTION

As used herein, the term "alkyl" is defined as straight chained and/or branched hydrocarbon groups containing the indicated number of carbon atoms and an open valence by which the alkyl group is attached to a base molecule; typical examples include methyl, ethyl, and straight chain and branched propyl and butyl groups. The hydrocarbon group can contain up to 16 carbon atoms, unless otherwise specified, and often contain one to eight carbon atoms. The term "alkyl" includes cycloalkyl and "bridged alkyl," *i.e.,* a C6-C16 bicyclic or polycyclic hydrocarbon group, for example, norbornyl, adamantyl, bicyclo[2.2.2]octyl, bicyclo[2.2.1]heptyl, bicyclo[3.2.1]octyl, or decahydronaphthyl. The term "cycloalkyl" is defined as a cyclic C3-C8 hydrocarbon group, *e.g*., cyclopropyl, cyclobutyl, cyclohexyl, and cyclopentyl.

The term "alkenyl" is defined identically as "alkyl," except the alkenyl group contains at least one carbon-carbon double bond, and as a result, the minimum size for an alkenyl group is C2. "Alkynyl" is defined similarly, except that an alkynyl group contains at least one carbon-carbon triple bond.

"Cycloalkenyl" is defined similarly to cycloalkyl, except at least one carbon-carbon double bond is present in the ring.

The term "alkylene" is defined as an alkyl group having a second open valence to which another group is attached, *i.e.,* an alkylene must connect two other substructures. For example, the term "aryl-C1-C3-alkylene" refers to an alkylene group containing one to three carbon atoms, and substituted at one valence with an aryl group, which leaves one remaining valence of the alkylene portion of the group as the point at which it is connected to a base molecule.

The terms "heteroalkyl", "heteroalkenyl", "heteroalkynyl", and "heteroalkylene" as used herein are defined similarly to the terms alkyl, alkenyl, alkynyl and alkylene, except that the 'hetero' forms include at least one heteroatom selected from N, O and S as a replacement for one of the carbons of the corresponding alkyl, alkenyl, alkynyl or alkylene moiety. In these heteroforms, S can be further oxidized to S=O or -SO₂-, *i.e*., it can have one or more =O substituents. These groups include at least one carbon atom, and are typically linked to a base molecule through carbon rather than by the heteroatom. Examples of heteroalkyl include methoxymethyl and dimethylaminoethyl, for example, and -CH₂-SO₂-CH₂- is an example of a heteroalkylene.

The term "halo" or "halogen" is defined herein to include fluorine, bromine, chlorine, and iodine. Frequently, fluoro or chloro is preferred in the compounds of formula (1) and (2).

The term "aryl," alone or in combination, is defined herein as a monocyclic or polycyclic aromatic group, *e.g.,* phenyl or naphthyl. Unless otherwise indicated, an "aryl" group can be unsubstituted or substituted, for example, with one or more, and in particular one to three substituents. Preferred substituents for the aryl groups of the invention include halo, alkyl, phenyl, hydroxyalkyl, alkoxy, alkoxyalkyl, haloalkyl, nitro, and amino. Exemplary aryl groups include phenyl, naphthyl, biphenyl, tetrahydronaphthyl, chlorophenyl, fluorophenyl, aminophenyl, methylphenyl, methoxyphenyl, trifluoromethylphenyl, nitrophenyl, carboxyphenyl, and the like. Fluoro, chloro, CF₃, CN, methyl, methoxy, dimethylamino, amino, and amine-substituted alkyl and heteroalkyl groups are typical examples suitable as substituents for an aryl ring, whether it is a single ring or is fused to another aryl, nonaryl, or heteroaryl ring.

The term "heteroaryl" is defined herein as a monocyclic or bicyclic ring system containing one or two aromatic rings and containing at least one nitrogen, oxygen, or sulfur atom as a ring member of an aromatic ring, and which can be unsubstituted or substituted, for example, with one or more, and in particular one to three, substituents, like halo, alkyl, hydroxy, hydroxyalkyl, alkoxy, alkoxyalkyl, haloalkyl, nitro, and amino. F, Cl, NH₂, MeNH (methylamine), OMe, Me, and CF₃ as well as amine-substituted alkyl or heteroalkyl groups are often preferred substituents for the heteroaryl groups of the invention. Examples of heteroaryl groups include thienyl, furyl, pyridyl, oxazolyl, quinolyl, isoquinolyl, indolyl, triazolyl, isothiazolyl, isoxazolyl, imidazolyl, benzothiazolyl, pyrazinyl, pyrimidinyl, thiazolyl, and thiadiazolyl.

"Pharmaceutically acceptable salts" refer to any salt that is physiologically acceptable insofar as it is compatible with other ingredients of the formulation and not deleterious to the recipient thereof. Some specific preferred examples are: acetate, trifluoroacetate, hydrochloride, methanesulfonate, succinate, malonate, maleate, hydrobromide, sulfate, citrate, tartrate, glycolate, and oxalate salts, which may be formed when the compound includes a basic (protonatable) feature. Similarly, the pharmaceutically acceptable salts include base addition products when the compound of the invention includes an acidic (de-protonatable) feature. Non-limiting examples of counterions for the deprotonated compounds of the invention include sodium, magnesium, calcium, ammonium, potassium, lithium, zinc, and similar cations.

"Inflammatory disorder" as used herein can refer to any disease, disorder, or syndrome in which an excessive or unregulated inflammatory response leads to excessive inflammatory symptoms, host tissue damage, or loss of tissue function. "Inflammatory disorder" also refers to a pathological state mediated by influx of leukocytes and/or neutrophil chemotaxis. These disorders include, but are not limited to, conditions wherein excessive immune activity occurs, such as rheumatoid arthritis, systemic lupus erythematosus (SLE), asthma, and allergies.

"Inflammation" as used herein refers to a localized, protective response elicited by injury or destruction of tissues, which serves to destroy, dilute, or wall off (sequester) both the injurious agent and the injured tissue. Inflammation is notably associated with influx of leukocytes and/or neutrophil chemotaxis. Inflammation can result from infection with pathogenic organisms and viruses and from noninfectious means such as trauma or reperfusion following myocardial infarction or stroke, immune response to foreign antigen, and autoimmune responses. Accordingly, inflammatory disorders amenable to the invention encompass disorders associated with reactions of the specific defense system as well as with reactions of the nonspecific defense system.

As used herein, the term "specific defense system" refers to the component of the immune system that reacts to the presence of specific antigens. Examples of inflammation resulting from a response of the specific defense system include the classical response to foreign antigens, autoimmune diseases, and delayed type hypersensitivity response mediated by T-cells. Chronic inflammatory diseases, the rejection of solid transplanted tissue and organs, *e.g*., kidney and bone marrow transplants, and graft versus host disease (GVHD), are further examples of inflammatory reactions of the specific defense system.

The term "nonspecific defense system" as used herein refers to inflammatory disorders that are mediated by leukocytes that are incapable of immunological memory (*e.g*., granulocytes, and macrophages). Examples of inflammation that result, at least in part, from a reaction of the nonspecific defense system include inflammation associated with conditions such as adult (acute) respiratory distress syndrome (ARDS) or multiple organ injury syndromes; reperfusion injury; acute glomerulonephritis; reactive arthritis; dermatoses with acute inflammatory components; acute purulent meningitis or other central nervous system inflammatory disorders such as stroke; thermal injury; inflammatory bowel disease; granulocyte transfusion associated syndromes; and cytokine-induced toxicity.

"Selectively inhibits" as used herein refers to a compound that has a greater inhibitory activity against one isoform of PI3K, usually the delta isoform, than it has against at least one of the other isoforms. In the methods of the invention wherein a selective PI3Kδ inhibitor is employed, it is preferred that the compound be at least about 10-fold selective for inhibition of PI3Kδ relative to at least one of the other Type I PI3K isoforms (alpha, beta and gamma) in a cell-based assay. More preferably, the compound is at least about 20-fold selective for inhibition of PI3Kδ relative to at least one of the other Type I PI3K isoforms in a cell-based assay. Still more preferably, the compound is at least about 50-fold more active for inhibition of PI3Kδ relative to other Type I PI3K isoforms in a biochemical assay. A PI3Kδ selective inhibitor compound is therefore more selective for PI3Kδ than conventional PI3K inhibitors such as wortmannin and LY294002, which are "nonselective PI3K inhibitors."

As used herein, the term "aberrant proliferation" means cell proliferation that deviates from the normal, proper, or expected course. For example, aberrant cell proliferation may include inappropriate proliferation of cells whose DNA or other cellular components have become damaged or defective. Aberrant cell proliferation may include cell proliferation whose characteristics are associated with an indication caused by, mediated by, or resulting in inappropriately high levels of cell division, inappropriately low levels of apoptosis, or both. Such indications may be characterized, for example, by single or multiple local abnormal proliferations of cells, groups of cells, or tissue(s), whether cancerous or non-cancerous, benign or malignant.

As used herein, the term "hematopoietic cells" generally refers to blood cells including but not limited to lymphoid progenitor cells, myeloid progenitor cells, natural killer cells, T cells, B cells, plasma cells, erythrocytes, megakaryocytes, monocytes, macrophages, and granulocytes such as neutrophils, eosinophils, and basophils.

As used herein, the term "amount effective" or "effective amount" means a dosage sufficient to produce a desired or stated effect.

"Autoimmune disease" as used herein refers to any of a group of disorders in which tissue injury is associated with humoral or cell-mediated responses to the body's own constituents. Rheumatoid arthritis, multiple sclerosis, and lupus (SLE) are specific examples.

"Allergic disease" as used herein refers to any symptoms, tissue damage, or loss of tissue function resulting from excessive allergic response; asthma is often a result of such allergic response. "Arthritic disease" as used herein refers to any disease that is characterized by inflammatory lesions of the joints attributable to a variety of etiologies. "Dermatitis" as used herein refers to any of a large family of diseases of the skin that are characterized by inflammation of the skin attributable to a variety of etiologies. "Transplant rejection" as used herein refers to any immune reaction directed against grafted tissue, such as organs or cells (*e.g*., bone marrow), characterized by a loss of function of the grafted and surrounding tissues, pain, swelling, leukocytosis, and thrombocytopenia.

"Treating" as used herein refers to preventing a disorder from occurring in an animal that can be predisposed to the disorder, but has not yet been diagnosed as having it; inhibiting the disorder, *i.e*., arresting its development; relieving the disorder, *i.e*., causing its regression; or ameliorating the disorder, *i.e*., reducing the severity of symptoms associated with the disorder. "Disorder" is intended to encompass medical disorders, diseases, conditions, syndromes, and the like, without limitation.

The selective PI3Kδ inhibitors of the present invention are compounds of formula (1): 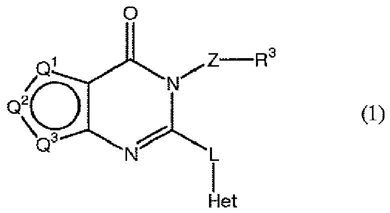 wherein:
one of Q¹, Q² and Q³ is S, and the other of two of Q¹, Q² and Q³ are -CR¹-;
   wherein each R¹ is independently H, halo, OR, NR₂, NROR, NRNR₂, SR, SOR, SO₂R, SO₂NR₂, NRSO₂R, NRCONR₂, NRCOOR, NRCOR, CF₃, CN, COOR, CONR₂, OOCR, COR, or NO₂,
   or R¹ can be an optionally substituted member selected from the group consisting of C1-C8 alkyl, C2-C8 heteroalkyl, C2-C8 alkenyl, C2-C8 heteroalkenyl, C2-C8 alkynyl, C2-C8 heteroalkynyl, C1-C8 acyl, C2-C8 heteroacyl, C6-C10 aryl, C5-C12 heteroaryl, C7-C12 arylalkyl, and C6-C12 heteroarylalkyl groups,
   wherein each R is independently H or C1-C8 alkyl, C2-C8 heteroalkyl, C2-C8 alkenyl, C2-C8 heteroalkenyl, C2-C8 alkynyl, C2-C8 heteroalkynyl, C1-C8 acyl, C2-C8 heteroacyl, C6-C10 aryl, C5-C10 heteroaryl, C7-C12 arylalkyl, or C6-C12 heteroarylalkyl,
   and wherein two R on the same atom or on adjacent atoms can be linked to form a 3-8 membered ring, optionally containing one or two N, O or S as ring members;
   and wherein each R group other than H, and each ring formed by linking two R groups together, is optionally substituted;
Z is a bond, or is O, NR², C1-C6 alkylene or C1-C6 heteroalkylene, each of which is optionally substituted with up to two C1-C6 alkyl or C2-C6 heteroalkyl groups, where two of said alkyl or heteroalkyl groups can optionally cyclize to form a 3-7 membered ring containing up to two heteroatoms selected from O, N and S as ring members;
R³ is aryl, heteroaryl, cycloalkyl, or heterocycloalkyl, each of which is optionally substituted with up to three R¹,
   or R³ can be H if Z is not a bond;
L is selected from the group consisting of -C(R²)₂, -C(R²)₂-C(R²)₂-, -C(R²)₂-NR²- , and -C(R²)₂-S(O)ₙ-,
   wherein each R² is independently H or an optionally substituted member selected from C1-C6 alkyl, C2-C6 heteroalkyl, C2-C6 alkenyl, and C2-C6 alkynyl, and n is 0-2;
   and two R², if present on L, can cyclize to form a 3-7 membered ring that may contain up to two heteroatoms selected from N, O and S as ring members;
Het is a monocyclic or bicyclic ring system wherein at least two ring atoms are N and wherein at least one ring is aromatic, and Het is optionally substituted with up to three substituents selected from R⁴, N(R⁴)₂, S(O)ₚR⁴, OR⁴, halo, CF₃, CN, NR⁴OR⁴, NR⁴N(R⁴)₂, SR⁴, SOR⁴, SO₂R⁴, SO₂N(R⁴)₂, NR⁴SO₂R⁴, NR⁴CON(R⁴)₂, NR⁴COOR⁴, NR⁴COR⁴, CN, COOR⁴, CON(R⁴)₂, OOCR⁴, COR⁴, or NO₂,
   wherein each R⁴ is independently H or an optionally substituted member selected from the group consisting of C1-C8 alkyl, C2-C8 heteroalkyl, C2-C8 alkenyl, C2-C8 heteroalkenyl, C2-C8 alkynyl, C2-C8 heteroalkynyl, C1-C8 acyl, C2-C8 heteroacyl, C6-C10 aryl, C5-C10 heteroaryl, C7-C12 arylalkyl, and C6-C12 heteroarylalkyl,
   and wherein two R⁴ on the same atom or on adjacent atoms can be linked to form a 3-8 membered ring, optionally containing one or two heteroatoms selected from N, O and S;
   wherein the optional substituents on each optionally substituted alkyl, heteroalkyl, alkenyl, heteroalkenyl, alkynyl, heteroalkynyl, acyl, heteroacyl, aryl, heteroaryl, arylalkyl and heteroarylalkyl are selected from C1-C4 alkyl, halo, CF₃, CN, =O, =N-CN, =N-OR', =NR', OR', NR'₂, SR', SO₂R', SO₂NR'₂, NR'SO₂R', NR'CONR'₂, NR'COOR', NR'COR', CN, COOR', CONR'₂, OOCR', COR', and NO₂,
   wherein each R' is independently H, C1-C6 alkyl, C2-C6 heteroalkyl, C1-C6 acyl, C2-C6 heteroacyl, C6-C10 aryl, C5-C10 heteroaryl, C7-12 arylalkyl, or C6-12 heteroarylalkyl, each of which is optionally substituted with one or more groups selected from halo, C1-C4 alkyl, C1-C4 heteroalkyl, C1-C6 acyl, C1-C6 heteroacyl, hydroxy, amino, and =O;
   and wherein two R' on the same or adjacent atoms can be linked to form a 3-7 membered ring optionally containing up to three heteroatoms selected from N, O and S; and
   p is 0-2;
or a pharmaceutically acceptable salt thereof.

In certain embodiments of the invention, Q¹ is S and Q² and Q³ each represent CR¹. In other embodiments of the invention, Q² is S and Q¹ and Q³ each represent CR¹. In still other embodiments, Q³ is S and Q¹ and Q² each represent CR¹. At least one of Q¹, Q² and Q³ is often CH, and in some of these embodiments, two of them are CH. Where R¹ in Q¹, Q² or Q³ is other than H, it is frequently C1-C4 alkyl, CF₃, CN, or halo, or it may be an amine-substituted alkyl or heteroalkyl group as described below.

In some embodiments, R¹ on the thiophene ring represents an amine-substituted alkyl or heteroalkyl group such as -(CH₂)ₚ-NR'₂ or-O-(CH₂)ₚ-NR'₂ or -NR'-(CH₂)ₚ-N(R')₂, wherein p is 1-A and each R' is H or C1-C4 alkyl, and wherein two R' present on one N may cyclize to form a 3-8 membered ring, which can optionally include an additional heteroatom selected from N, O and S. specific examples of these amine-substituted alkyl and heteroalkyl groups include, without limitation:

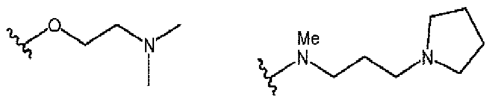

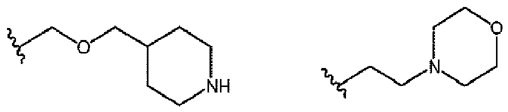

In some embodiments, Z represents a bond; in other embodiments, it represents (CH₂)₁₋₄. Where Z is a bond, R³ often represents an aryl or heteroaryl ring; in some of these embodiments, R³ is an optionally substituted phenyl or pyridyl ring. In these embodiments, R³ is often substituted with at least one substituent, which is frequently ortho or meta to the point of attachment of the aryl ring to Z. In some of these embodiments, R³ is substituted with 1-3 substituents such as halo, CN, methyl, CF₃, or an amine-substituted alkyl or heteroalkyl as described above for R¹. When Z is a bond, R³ is frequently phenyl, halo-substituted phenyl, dihalo phenyl, or cyanophenyl. In other embodiments, Z is a bond or (CH₂)₁₋₂, and R³ represents a cycloalkyl group, which can be substituted.

In some preferred embodiments of the compounds of formula (1), L is C(R²)₂ or C(R²)₂NH or C(R²)₂S, where each R² is independently H or C1-C4 alkyl, C2-C4 alkenyl or C2-C4 alkynyl. In certain embodiments, L is CH(R²) or CH(R²)NH or CH(R²)S, where R² represents methyl or ethyl or any optionally substituted C1-C6 alkyl group, or R² can be an amine-substituted alkyl group as described above for R¹. Where one terminus of L is a heteroatom, L is often linked to Het via this heteroatom; and the heteroatom of L is typically attached to a carbon atom of Het.

In many embodiments, the center CH(R²) of the linker "L" is chiral, and frequently the *S* enantiomer of this stereocenter is preferred. In other embodiments, this stereocenter is an *R* enantiomer. Typically, where L is CH(R²)NH or CH(R²)S, the N or S is linked to Het, and CH(R²) is directly bonded to the pyrimidinone ring. In still other embodiments, L is -CH(R²)-NR²-, wherein the two R² groups are linked to form a ring, which is often a 5-6 atom ring. In these cyclic linkers, a chiral center is also present at CH(R²), and that center may be in either the R or S configuration; the S enantiomer is often preferred.

Het is an optionally substituted monocyclic or bicyclic ring, and at least one ring of Het is typically a heteroaryl ring containing at least two nitrogen atoms as ring members; in many embodiments, Het is a bicyclic aromatic heterocycle. In certain embodiments of the compounds of formula (1), Het represents a purine ring, which may be substituted with a C1-C4 alkyl group, C5-C10 aryl group, C5-C10 heteroaryl group, halo, amine, alkylamine, dialkylamine, or an amine-substituted alkyl or heteroalkyl group as described above for R¹. In other embodiments, Het represents a pyrazolopyrimidine or a pyrrolopyrimidine ring, each of which can be similarly substituted. In still other embodiments, Het represents a pyrimidine or triazine ring, which can also be similarly substituted.

Where Het represents a bicyclic group, it can be attached to L at any available ring position of Het. In many embodiments, L is attached to a carbon or nitrogen atom that is adjacent to an atom shared by both rings of the bicyclic group. In many embodiments, Het represents a 6, 5-bicyclic heteroaromatic group, and L may be attached to either the 5-membered ring or the 6-membered ring. In some embodiments, Het is a purine ring, for which the following atom numbering convention is used:

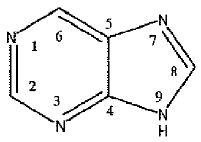

Some specific examples of Het, without limiting its scope, include the following, where [L] indicates the point at which Het is attached to linker "L", and - X represents a preferred point of attachment for a substituent when one is present. In some embodiments, no substituents other than H are present (each X represents H); in others where more than one X is shown, at least one X is H. In many embodiments, each X that is not H represents an amine or substituted amine, an alkyl or aryl group, or a halogen. Some preferred groups for X include NH₂, F, Cl, Me, CF₃, and phenyl.

Where L is attached at N-9 of a purine or purine analog (using the purine numbering scheme for simplicity, even when the ring is a purine analog), such as the heterocycles depicted below, L represents CH₂ or CH(R²). Where L is attached to C-6 of a purine or purine analog such as the heterocycles depicted here, L is frequently CH(R²)-NH, CH(R²)-S or CH(R²)-N(R²), and the heterocycle represented by Het is typically linked to the heteroatom of L in these embodiments. In many of these embodiments, R² on a carbon atom of the linker L is methyl or ethyl, and when R² is on nitrogen of the linker, it is often H.

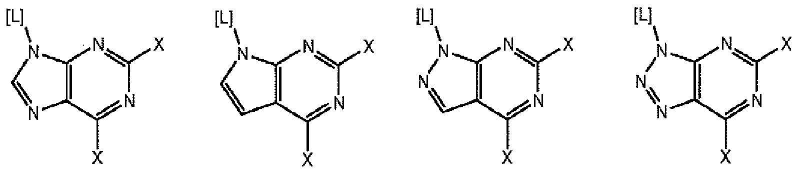

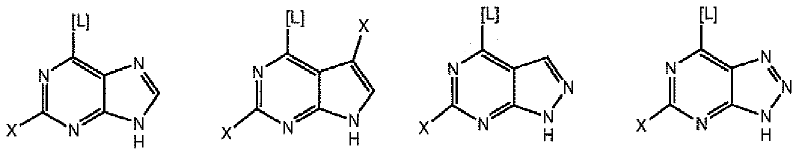

In some preferred embodiments of the compound of formula (1), R³ represents optionally substituted phenyl and Z is a bond. Particularly preferred phenyl groups include unsubstituted phenyl, 2-methylphenyl, 3-methylphenyl, 4-methylphenyl, 2-methoxyphenyl, 3-methoxyphenyl, 4-methoxyphenyl, 2-fluorophenyl, 3-fluorophenyl, 4-fluorophenyl, 2,3-difluorophenyl, 2,5-difluorophenyl, 2,4-difluorophenyl, 3,4-difluorophenyl, 3,5-difluorophenyl, and 2,6-difluorophenyl.

In some preferred embodiments of the compounds of formula (1), -L-Het represents -CH₂-Het, -CH₂-NH-Het, -CH₂-S-Het, -CHMe-Het, -CHMe-S-Het, - CHMe-NH-Het, -CHEt-Het, -CHEt-S-Het, or -CHEt-NH-Het.

In some preferred embodiments of the compounds of formula (1), Het represents a purine that is linked to L at position 6. In other preferred embodiments, when -L-Het is -CH₂-Het, -CHMe-Het, or -CHEt-Het, Het is purine that is linked to L at position 9 of the purine. When Het is a purine ring, it is sometimes substituted by amino, fluoro, methyl or CF₃; and sometimes it is unsubstituted. In other preferred embodiments, Het is a pyrazolopyrimidine and is linked to L at a position that corresponds to position 6 or position 9 of the purine ring, when the pyrimidine rings of the pyrazolopyrimidine and purine are overlaid for purposes of labeling the ring positions.

Compounds of formula (1) having any combination of the preferred features described above are sometimes particularly preferred.

Frequently, the PI3Kδ selective inhibitor may be a compound having formula (2a) or (2b) or (2c) or a pharmaceutically acceptable salt or solvate thereof: 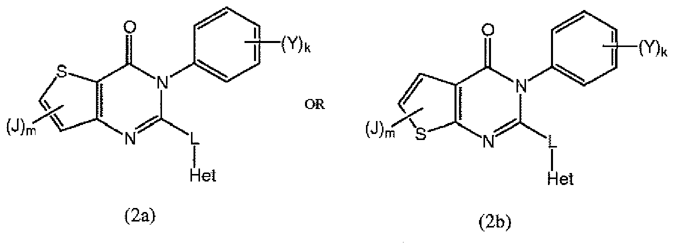 OR 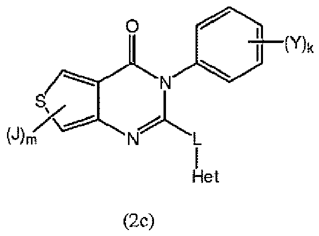 wherein:
each J and each Y is independently selected from the group consisting of F, Cl, Br, CN, Me, CF₃, OMe, CONR²₂, COOR², NMe₂, NH₂, NHMe, -Q-(CH₂)_{q}-OR², and -Q-(CH₂)_{q}-N(R²)₂, where q is 0-4, and Q is absent or is selected from O, S and NR²;
m is 0-2, and k is 0-3;
L is selected from -C(R²)₂-, -C(R²)₂-NR²-, and -C(R²)₂-S-,
each R² is independently H or an optionally substituted C1-C4 alkyl, C2-C4 alkenyl, or C2-C4 alkynyl, or an optionally substituted C2-C4 heteroalkyl;
   and two R², if present on a single atom or on adjacent atoms, can cyclize to form a 3-7 membered ring that is optionally substituted and may contain up to two heteroatoms selected from N, O and S as ring members;
Het is selected from the group consisting of: 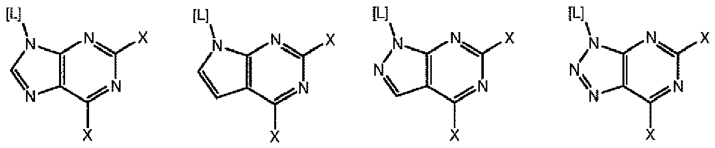 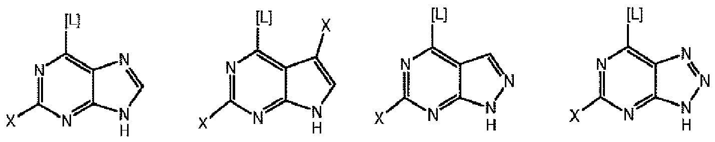 wherein [L] indicates the atom of Het to which L is attached; and
each X is independently H, F, Cl, Br, Me, CF₃, OH, OMe, NH₂, NHAc, or NHMe; and the optional substituents on each optionally substituted alkyl, heteroalkyl, alkenyl, heteroalkenyl, alkynyl, heteroalkynyl, acyl, heteroacyl, aryl, heteroaryl, arylalkyl and heteroarylalkyl are selected from C1-C4 alkyl, halo, =O, =N-CN, =N-OR', =NR', OR', NR'₂, SR', SO₂R', SO₂NR'₂, NR'SO₂R', NR'CONR'₂, NR'COOR', NR'COR', CN, COOR', CONR'₂, OOCR', COR', and NO₂,
   wherein each R' is independently H, C1-C6 alkyl, C2-C6 heteroalkyl, C1-C6 acyl, C2-C6 heteroacyl, C6-C10 aryl, C5-C10 heteroaryl, C7-12 arylalkyl, or C6-12 heteroarylalkyl, each of which is optionally substituted with one or more groups selected from halo, C1-C4 alkyl, C1-C4 heteroalkyl, C1-C6 acyl, C1-C6 heteroacyl, hydroxy, amino, and =O;
   and wherein two R' on the same or adjacent atoms can be linked to form a 3-7 membered ring optionally containing up to three heteroatoms selected from N, O and S;
   and p is 0-2;
or a pharmaceutically acceptable salt thereof.

The compounds of formula (2), which includes formula (2a) and formula (2b) and formula (2c), are preferred compounds within the scope of the invention. In these compounds, m is frequently 0 or 1, and J, if present, is frequently F, Cl or CF₃. Each Y is independently selected, and at least one Y often represents Me, OMe, CN, CF₃, or halo. In certain embodiments, Y is F, Me or CN. Each X for the Het group in formula (2) is independently selected, and frequently each X is H, F, Cl, Me, CF₃, phenyl, or NH₂.

In some preferred embodiments of the compound of formula (2), the phenyl ring shown is an selected from unsubstituted phenyl, 2-methylphenyl, 3-methylphenyl, 4-methylphenyl, 2-methoxyphenyl, 3-methoxyphenyl, 4-methoxyphenyl, 2-fluorophenyl, 3-fluorophenyl, 4-fluorophenyl, 2,3-difluorophenyl, 2,5-difluorophenyl, 2,4-difluorophenyl, 3,4-difluorophenyl, 3,5-difluorophenyl, and 2,6-difluorophenyl.

In some preferred embodiments of the compounds of formula (2), -L-Het represents -CH₂-Het, -CH₂-NH-Het, -CH₂-S-Het, -CHMe-Het, -CHMe-S-Het, - CHMe-NH-Het, -CHEt-Het, -CHEt-S-Het, or -CHEt-NH-Het.

In some preferred embodiments of the compounds of formula (2), Het represents a purine that is linked to L at position 6. In other preferred embodiments, when -L-Het is -CH₂-Het, -CHMe-Het, or -CHEt-Het, Het is purine that is linked to L at position 9 of the purine. When Het is a purine ring, it is sometimes unsubstituted, and it is sometimes substituted by amino, fluoro, aryl, methyl or CF₃; and sometimes it is unsubstituted. In other preferred embodiments, Het is a pyrazolopyrimidine and is linked to L at a position that corresponds to position 6 or position 9 of the purine ring, when the pyrimidine rings of the pyrazolopyrimidine and purine are overlaid for purposes of labeling the ring positions.

Where a stereocenter is present in a compound of formula (1) or formula (2), the compounds and methods include a racemic mixture of the compound of the invention or either specific enantiomer. If more than one stereocenter is present, any mixture of isomers, including racemic forms of each individual diastereomer, may be used. In preferred embodiments where a stereocenter is present in the linking group "L" in formula (1) or (2), the S-enantiomer of that stereocenter is often used. However, the compounds and methods of the invention include each possible stereoisomer and geometric isomer of the aforementioned compounds, and mixtures containing two or more isomers of these compounds.

In certain embodiments, the compounds and methods include PI3Kδ selective inhibitors of formula (1) or (2) containing an amine-substituted alkyl or heteroalkyl group that improve solubility properties, such as by providing for formation of salts. This amine-substituted alkyl or heteroalkyl group may be attached to any of the rings of these compounds, including Het or the linker "L" in formula (1), or it may be on Z or R³ in formula (1). The amine-substituted groups provide improved solubility characteristics for these compounds, and thus improve their pharmacokinetic properties without adversely affecting their selectivity for the delta isoform of PI3K. Suitable amine-substituted groups that may be present as substituents on the compounds of the invention include -(CH₂)ₚ-NR'₂ and -O-(CH₂)ₚ-NR'₂, wherein p is 1-4 and each R' is H or C1-C4 alkyl (frequently Me), and wherein two R' present on one N may cyclize to form a 3-8 membered ring, which can optionally include an additional heteroatom such as N, O or S.

The compounds of the invention are readily prepared from available starting material using methods that are known in the art. Examples of methods for constructing the thienopyrimidinone portion of the compounds of formula (1) and (2) are provided, for example, in published PCT application WO 03/050064. Scheme A in that application provides a route by which the thienopyrimidinone portions of compounds of formula (2a) can be prepared, and Schemes B-G provide routes by which the thienopyrimidinone portions of compounds of formula (2b) can be prepared. Compounds of formula (2c) can similarly be prepared from the available starting material corresponding to the appropriate thiophene isomer of compound B 1. Methods to convert the protected amines attached to position 2 of the pyrimidinone ring of these thienopyrimidinone intermediates are known in the art, and can be found, for example, in U.S. Patent Nos. 6,518,277; 6,667,300; 6,949,535; and 6,800,620, and in published U.S. Patent Application US 2006/0106038 and PCT application WO 2005/113554. These references also provide methods for determining the activity of these compounds as inhibitors of PI3Kδ; thus those methods are known in the art.

The following Schemes illustrate preparation of selected compounds within the scope of the invention.

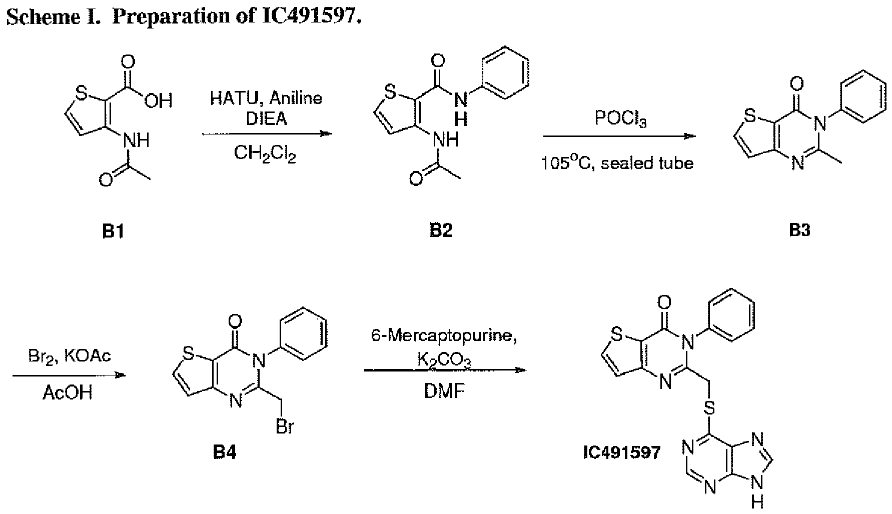

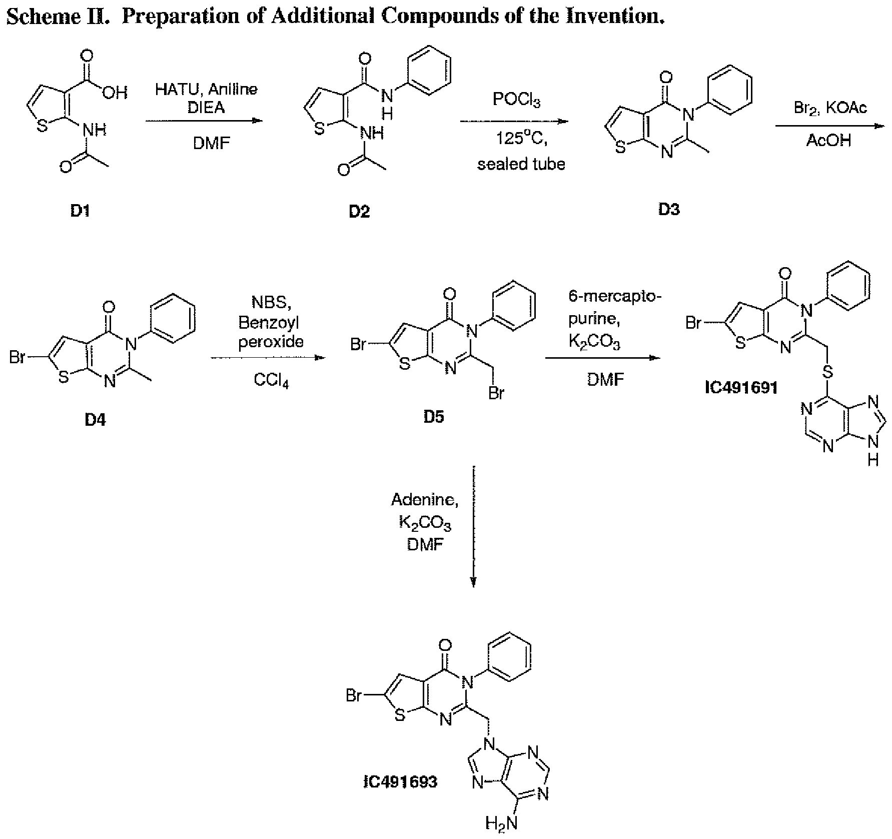

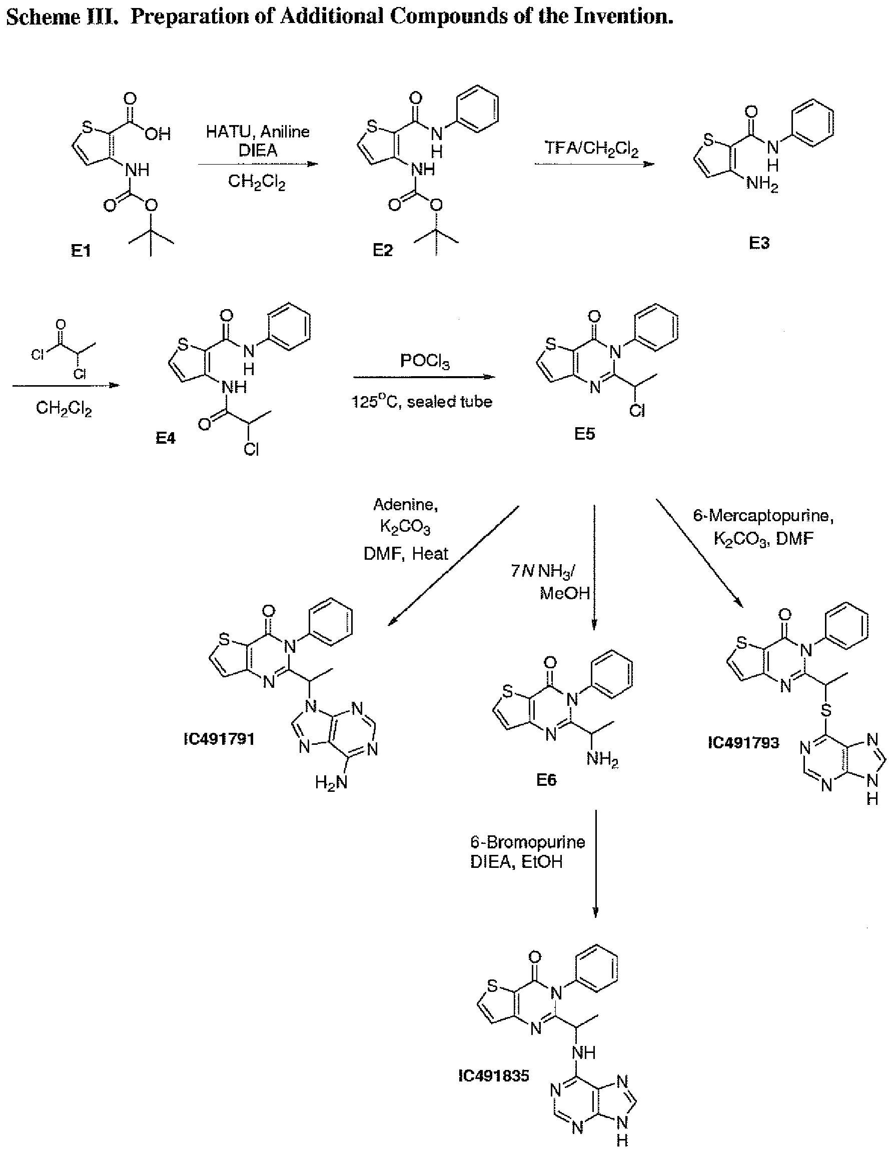

Intermediates B4, D5 and E5 can be used to alkylate various known materials to introduce other Het groups. An example of this method is provided herein in Example 16.

Preferred embodiments of the invention include those depicted in the foregoing Schemes, and the accompanying Examples. Exemplary compounds include (3a) and (3b) for which comparative activity data is provided in Table I (relative to reference compounds (4a) and (4b)):

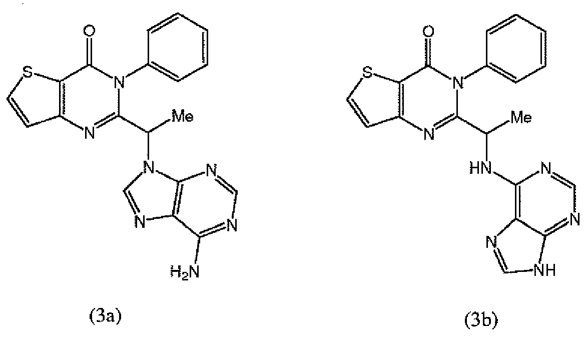

The S-enantiomers of these compounds are particularly preferred embodiments.

In some embodiments of the invention, in a compound of formula (1) or (2), Het is a purine that is linked to L at either position 9 (N-9 of the purine) or position 6 (C-6 of the purine). Compound (3a) is an example of a compound of the invention wherein Het is a purine that is linked to the linking group at purine position N-9, and compound (3b) is an example of a compound where Het is a purine that is linked to the linking group L at purine position C-6. Each of these compounds is highly active, and each is highly selective for inhibition of PI3Kδ relative to its activity on other PI3K isoforms, as illustrated in the following table, where these two compounds are compared to other PI3K inhibitors (reference compounds 4a and 4b) having similar purine and linking components:

| Ref. No. | COMPOUND | Ki (nM) (PI3Kδ) | Selectivity (α/δ) | Selectivity (β/δ) | Selectivity (γ/δ) |
|---|---|---|---|---|---|
| 3a | 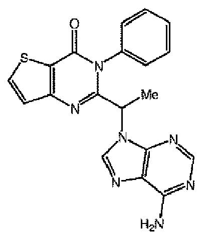 | 40 | 1447 | 126 | 187 |
| 4a | 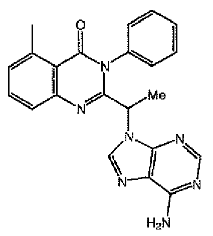 | 7 | 2963 | 119 | 61 |
| 3b | 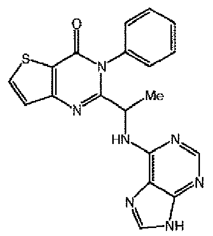 | 6 | 528 | 123 | 28 |
| 4b | 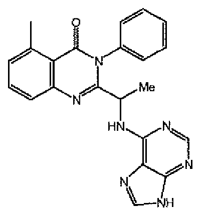 | 5 | 132 | 29 | 12 |

The methods of the invention embrace various modes of treating an animal subject, preferably a mammal, more preferably a primate, and still more preferably a human. Among the mammalian animals that can be treated are, for example, companion animals (pets), including dogs and cats; farm animals, including cattle, horses, sheep, pigs, and goats; laboratory animals, including rats, mice, rabbits, guinea pigs, and nonhuman primates, and zoo specimens. Non-mammalian animals include, for example, birds, fish, reptiles, and amphibians.

As previously described, the term "PI3Kδ selective inhibitor" generally refers to a compound that inhibits the activity of the PI3Kδ isozyme more effectively than other isozymes of the PI3K family. The relative efficacies of compounds as inhibitors of an enzyme activity (or other biological activity) can be established by determining the concentrations at which each compound inhibits the activity to a predefined extent and then comparing the results. Typically, the preferred determination is the concentration that inhibits 50% of the activity in a biochemical assay, i.e., the 50% inhibitory concentration or "TC₅₀"- IC₅₀ determinations can be accomplished using conventional techniques known in the art. In general, an IC₅₀ can be determined by measuring the activity of a given enzyme in the presence of a range of concentrations of the inhibitor under study. The experimentally obtained values of enzyme activity then are plotted against the inhibitor concentrations used. The concentration of the inhibitor that shows 50% enzyme activity (as compared to the activity in the absence of any inhibitor) is taken as the IC₅₀ value. Analogously, other inhibitory concentrations can be defined through appropriate determinations of activity. For example, in some settings it can be desirable to establish a 90% inhibitory concentration, *i.e*., IC₉₀, etc.

Accordingly, a PI3Kδ selective inhibitor alternatively can be understood to refer to a compound that exhibits a 50% inhibitory concentration (IC₅₀) with respect to PI3Kδ that is at least 10-fold, in another aspect at least 20-fold, and in another aspect at least 30-fold, lower than the IC₅₀ value with respect to any or all of the other Class I PI3K family members. In an alternative embodiment of the invention, the term PI3Kδ selective inhibitor can be understood to refer to a compound that exhibits an IC₅₀ with respect to PI3Kδ that is at least 50-fold, in another aspect at least 100-fold, in an additional aspect at least 200-fold, and in yet another aspect at least 500-fold, lower than the IC₅₀ with respect to any or all of the other PI3K Class I family members. A PI3Kδ selective inhibitor is typically administered in an amount such that it selectively inhibits PI3Kδ activity, as described above.

The methods of the invention may be applied to cell populations *in vivo* or *ex vivo*. "*In vivo*" means within a living individual, as within an animal or human. In this context, the methods of the invention may be used therapeutically in an individual, as described *infra.* The methods may also be used prophylactically including but not limited to when certain risk factors associated with a given indication treatable by the methods of the invention are present, particularly when two or more such risk factors are present. Many such risk factors are related to an individual's risk of relapse. Individuals having a high risk of relapse include but are not limited to individuals having chromosomal abnormalities involving chromosomes 3, 5, and/or 7. Other risk factors include but are not limited to the following: having a close relative who has been diagnosed with an indication involving aberrant proliferation of hematopoietic cells; having Down's syndrome or other disease caused by abnormal chromosomes; repeated or substantial exposure to benzene and/or other organic solvents; exposure to high doses of ionizing radiation; having received treatments comprising certain chemotherapeutic agents; exposure to diagnostic X-rays during pregnancy; infection with human T-cell leukemia virus; and, cigarette smoking and/or substantial exposure to smoke. Additional risk factors that may indicate that prophylactic treatment is warranted are known in the art and/or may be readily determined by the attending physician.

"*Ex vivo*" means outside of a living individual. Examples of *ex vivo* cell populations include *in vitro* cell cultures and biological samples including but not limited to fluid or tissue samples obtained from individuals. Such samples may be obtained by methods well known in the art. Exemplary biological fluid samples include blood, cerebrospinal fluid, urine, saliva. Exemplary tissue samples include tumors and biopsies thereof. In this context, the invention may be used for a variety of purposes, including therapeutic and experimental purposes. For example, the invention may be used *ex vivo* to determine the optimal schedule and/or dosing of administration of a PI3Kδ selective inhibitor for a given indication, cell type, individual, and other parameters. Information gleaned from such use may be used for experimental purposes or in the clinic to set protocols for *in vivo* treatment. Other *ex vivo* uses for which the invention may be suited are described below or will become apparent to those skilled in the art.

PI3Kδ is primarily expressed in hematopoietic cells; consequently, the direct effects of selective inhibitors of PI3Kδ are most apparent in hematopoietic cells. Hematopoietic cells typically differentiate into either lymphoid progenitor cells or myeloid progenitor cells, both of which ultimately differentiate into various mature cell types including but not limited to leukocytes. Aberrant proliferation of hematopoietic cells of one type often interferes with the production or survival of other hematopoietic cell types, which can result in compromised immunity, anemia, and/or thrombocytopenia. The methods of the invention treat and/or prevent aberrant proliferation of hematopoietic cells by inhibiting aberrant proliferation of hematopoietic cells. As a result, they may also ameliorate the symptoms and secondary conditions that result from a primary effect such as excessive system or localized levels of leukocytes or lymphocytes.

Various disease states, disorders, and conditions (hereafter, indications) involving aberrant proliferation of hematopoietic cells (including excessive production of lymphoid progenitor cell-derived cells and/or myeloid progenitor cell-derived cells) include but are not limited to leukemias, lymphomas, myeloproliferative disorders, myelodysplastic syndromes, and plasma cell neoplasms.

In one aspect, the invention provides methods for treating and/or preventing aberrant proliferation of hematopoietic cells comprising selectively inhibiting phosphoinositide 3-kinase delta (PI3Kδ) activity in hematopoietic cells, using a compound of formula (1) or formula (2). Thus, in one aspect, the methods comprise administering an amount of a PI3Kδ selective inhibitor effective to inhibit PI3Kδ activity in hematopoietic cells.

The methods of the invention may generally be used to treat and/or prevent indications involving aberrant proliferation of hematopoietic cells. Accordingly, the methods may be used to treat and/or prevent indications involving aberrant proliferation of lymphoid and/or myeloid progenitor cells including but not limited to leukemias such as acute lymphoblastic leukemia, acute myeloid leukemia; chronic lymphocytic leukemia, chronic myelogenous leukemia, and hairy cell leukemia; myeloproliferative disorders such as polycythemia vera, chronic idiopathic myelofibrosis, and essential thrombocythemia; myelodysplastic syndromes such as refractory anemia, refractory anemia with ringed sideroblasts, refractory anemia with excess blasts, and refractory anemia with excess blasts in transformation; lymphomas such as Hodgkin's lymphoma and non-Hodgkin's lymphomas such as B-cell lymphoma, Burkitt's lymphoma, diffuse cell lymphoma, follicular lymphoma, immunoblastic large cell lymphoma, lymphoblastic lymphoma, mantle cell lymphoma, mycosis fungoides, post-transplantation lymphoproliferative disorder, small non-cleaved cell lymphoma, and T-cell lymphoma; and, plasma cell neoplasms such as myelomas.

In one embodiment, the invention provides methods for treating and/or preventing leukemia comprising selectively inhibiting phosphoinositide 3-kinase delta (PI3Kδ) activity in leukemic cells. In one aspect of this embodiment, the methods comprise administering an amount of a PI3Kδ selective inhibitor effective to inhibit PI3Kδ activity of hematopoietic cells.

As used herein, the term "leukemia" generally refers to cancers that are characterized by an uncontrolled increase in the number of at least one leukocyte and/or leukocyte precursor in the blood and/or bone marrow. Leukemias including but not limited to acute lymphoblastic leukemia (ALL); acute myeloid leukemia (AML); chronic lymphocytic leukemia (CLL); chronic myelogenous leukemia (CML); and, hairy cell leukemia are contemplated. "Leukemic cells" typically comprise cells of the aforementioned leukemias.

The PI3K pathway is constitutively activated in the aberrantly proliferating hematopoietic cells. In one aspect of this embodiment, a higher level of phosphorylated Akt protein is present in untreated aberrantly proliferating hematopoietic cells relative to normal hematopoietic cells (*i.e*., non-aberrantly proliferating hematopoietic cells). In an additional aspect, a higher level of phosphorylated FOXO3a protein is present in untreated hematopoietic cells, and/or a higher level of phosphorylated GAB 1 protein or phosphorylated GAB 2 protein is present in untreated hematopoietic cells, in each instance relative to normal hematopoietic cells.

Thus, in one aspect, the PI3Kδ selective inhibitor is administered in an amount effective to inhibit Akt phosphorylation in aberrantly proliferating hematopoietic cells. In another aspect, the PI3Kδ selective inhibitor is administered in an amount effective to inhibit FOXO3a phosphorylation in aberrantly proliferating hematopoietic cells. In a further aspect, the PI3Kδ selective inhibitor is administered in an amount effective to inhibit GAB1 phosphorylation and/or GAB2 phosphorylation in aberrantly proliferating hematopoietic cells.

Animal models of some of the foregoing indications involving aberrant proliferation of hematopoietic cells treatable by selective inhibitors of PI3Kδ, such as the compounds of the invention, include, for example: non-obese diabetic-severe combined immune deficient (NOD/scid) mice injected with human ALL cells (ALL model); athymic (rnu/rnu) nude rats injected with human ALL cells (*e.g*., HPB-ALL cells) (ALL model); NOD/scid mice injected with human CML cells (CML model); inbred Sprague-Dawley/Charles University Biology (SD/Cub) rats (spontaneous T-cell lymphoma/leukemia model); Emu-immediate-early response gene X-1 (IEX-1) mice (T-cell lymphoma model); rabbits injected with cynomogulus-Epstein Barr virus (T-cell lymphoma model); rabbits injected with Herpes virus papio (T-cell lymphoma model); transgenic mice expressing p210bcr/ab1 (founder mice, ALL model; progeny mice, CML model); NOD/scid/gammac null (NOG) mice injected with U266 cells (multiple myeloma model); and, C57B1/KaLwRij mice injected with ST33 cells (multiple myeloma model).

Aberrant cell proliferation is cell proliferation that deviates from the normal, proper, or expected course. Aberrant cell proliferation is the hallmark of cancer. Cancers can generally be divided into solid tumors affecting organs and/or connective tissues (including but not limited to bone and cartilage) and hematological malignancies that arise from hematopoietic cells. Hematopoietic cells typically differentiate into either lymphoid progenitor cells or myeloid progenitor cells, both of which ultimately differentiate into various mature cell types including but not limited to leukocytes. Lymphoid progenitor cell-derived cells include but are not limited to natural killer cells, T cells, B cells, and plasma cells. Myeloid progenitor cell-derived cells include but are not limited to erythrocytes (red blood cells), megakaryocytes (platelet producing cells), monocytes, macrophages, and granulocytes such as neutrophils, eosinophils, and basophils. Because the aforementioned leukocytes are integral components of the body's immune system, aberrant proliferation of hematopoietic cells can impair an individual's ability to fight infection. Additionally, aberrant proliferation of hematopoietic cells of one type often interferes with the production or survival of other hematopoietic cell types, which can result in anemia and/or thrombocytopenia. Thus the inhibition of aberrant proliferation of hematopoietic cells is advantageous for the treatment of other conditions that may be associated with a hematopoietic cell cancer.

Leukemias are cancers that are characterized by an uncontrolled increase in the number of at least one type of leukocyte and/or leukocyte precursor in the blood and/or bone marrow. Leukemias are generally classified as either acute or chronic, which correlates with both the tempo of the clinical course and the degree of leukocyte differentiation. In acute leukemias, the involved cell line (usually referred to as blast cells) shows little or no differentiation. In chronic leukemias, on the other hand, the involved cell line is typically more well-differentiated but immunologically incompetent. Leukemias are also further classified according to cell lineage as either myelogenous (when myeloid progenitor cell-derived cells are involved) or lymphocytic (when lymphoid progenitor cell-derived cells are involved). Additionally, secondary leukemias can develop in patients treated with cytotoxic agents such as radiation, alkylating agents, and epipodophyllotoxins. Inhibitors of PI3Kδ reduce the rate of production of these hematopoietic cells, thus at least slowing the progression of leukemia in its various forms. Selective inhibitors such as the compound of formula (1) achieve this effect with minimal disruption to the functioning of other types of cells, which typically utilize other isoforms of PI3K.

Lymphomas are cancers that originate in lymphocytes of lymphoid tissues including but not limited to the lymph nodes, bone marrow, spleen, and other organs of the immune system, and are characterized by uncontrolled increase in lymphocyte production. There are two basic categories of lymphomas, Hodgkin's lymphoma, which is marked by the presence of a hallmark cell type called the Reed-Sternberg cell, and non-Hodgkin's lymphomas, which includes a large, diverse group of lymphocytic cancers. The non-Hodgkin's lymphomas are generally classified according to lymphocyte cell lineage (including but not limited to B cells, T cells, and natural killer cells), and can be further divided into cancers that have an indolent (slowly progressing or low grade) course and those that have an aggressive (rapidly progressing or intermediate or high grade) course. Non-Hodgkin's lymphomas include but are not limited to B-cell lymphoma, Burkitt's lymphoma, diffuse cell lymphoma, follicular lymphoma, immunoblastic large cell lymphoma, lymphoblastic lymphoma, mantle cell lymphoma, mycosis fungoides, post-transplantation lymphoproliferative disorder, small non-cleaved cell lymphoma, and T-cell lymphoma. Again, selective inhibitors of PI3Kδ reduce the growth rate of lymphomas by their selective activity against hematopoietic cells.

Myeloproliferative disorders also involve excessive production of certain types of blood cells in the bone marrow. Myeloproliferative disorders include but are not limited to polycythemia vera, chronic idiopathic myelofibrosis, and essential thrombocythemia. In polycythemia vera, red blood cells are overproduced in the bone marrow and build up in the blood stream. In chronic idiopathic myelofibrosis, aberrant proliferation of myeloid progenitor-derived cells leads to fibrosis in the bone marrow and eventually bone marrow failure (*i.e*., an underproduction of myeloid progenitor-derived cells). In essential thrombocythemia, the number of platelets are overproduced, but other cells in the blood are normal. Selective inhibitors of PI3Kδ reduce the overproduction of these hematopoietic cells and thus ameliorate these conditions.

Myelodysplastic syndromes, sometimes referred to as pre-leukemias or "smoldering" leukemias, are additional indications in which the bone marrow does not function normally, a so called "ineffective hematopoiesis". Immature blast cells do not mature properly and become overproduced, leading to a lack of effective mature blood cells. A myelodysplastic syndrome may develop following treatment with drugs or radiation therapy for other diseases, or it may develop without any known cause. Myelodysplastic syndromes are classified based on the appearance of bone marrow and blood cells as imaged by microscope. Myelodysplastic syndromes include but are not limited to refractory anemia, refractory anemia with ringed sideroblasts, refractory anemia with excess blasts, and refractory anemia with excess blasts in transformation.

Plasma cell neoplasms including but not limited to myelomas are malignancies of bone marrow plasma cells that resemble leukemia. The malignant plasma cells, otherwise known as myeloma cells, accumulate in the bone marrow and, unlike typical leukemias, rarely enter the blood stream. This progressive accumulation of myeloma cells within the marrow disrupts normal bone marrow function (most commonly reflected by anemia), reduces white cell and platelet counts, causes damage to surrounding bone, and suppresses normal immune function (reflected by reduced levels of effective immunoglobulins and increased susceptibility to infection). Myeloma cells usually grow in the form of localized tumors (plasmacytomas). Such plasmacytomas can be single or multiple and confined within bone marrow and bone (medullary) or developed outside of bone in soft tissue (extramedullary plasmacytomas). When there are multiple plasmacytomas inside or outside bone, the indication is also called multiple myeloma.

Such indications are typically treated with one or more therapies including but not limited to surgery, radiation therapy, chemotherapy, immunotherapy, and bone marrow and/or stem cell transplantation. Combinations of these treatments are often utilized, and the compounds of the invention may be used in combination with surgical, radiation, immunotherapy, and bone marrow and/or stem cell transplantation methods.

Surgery involves the bulk removal of diseased tissue. While surgery can be effectively used to remove certain tumors, for example, breast, colon, and skin, it cannot be used to treat tumors located in areas that are inaccessible to surgeons. Additionally, surgery cannot typically be successfully used to treat non-localized cancerous indications including but not limited to leukemias and myelomas.

Radiation therapy involves using high-energy radiation from x-rays, gamma rays, neutrons, and other sources ("radiation") to kill rapidly dividing cells such as cancerous cells and to shrink tumors. Radiation therapy is well known in the art (Hellman, Cancer: Principles and Practice of Oncology, 248-275, 4th ed., vol. 1 (1993)). Radiation therapy may be administered from outside the body ("externalbeam radiation therapy"). Alternatively, radiation therapy can be administered by placing radioactive materials capable of producing radiation in or near the tumor or in an area near the cancerous cells. Systemic radiation therapy employs radioactive substances including but not limited to radiolabeled monoclonal antibodies that can circulate throughout the body or localize to specific regions or organs of the body. Brachytherapy involves placing a radioactive "seed" in proximity to a tumor. Radiation therapy is non-specific and often causes damage to any exposed tissues. Additionally, radiation therapy frequently causes individuals to experience side effects (such as nausea, fatigue, low leukocyte counts, etc.) that can significantly affect their quality of life and influence their continued compliance with radiation treatment protocols.

Chemotherapy involves administering chemotherapeutic agents that often act by disrupting cell replication or cell metabolism (e.g., by disrupting DNA metabolism, DNA synthesis, DNA transcription, or microtubule spindle function, or by perturbing chromosomal structural integrity by way of introducing DNA lesions). Chemotherapeutics are frequently non-specific in that they affect normal healthy cells as well as tumor cells. The maintenance of DNA integrity is essential to cell viability in normal cells. Chemotherapeutic agents must be potent enough to kill cancerous cells without causing too much damage to normal cells. Therefore, anticancer drugs typically have very low therapeutic indices, i.e., the window between the effective dose and the excessively toxic dose can be extremely narrow because the drugs cause a high percentage of damage to normal cells as well as tumor cells. Additionally, chemotherapy-induced side effects significantly affect the quality of life of an individual in need of treatment, and therefore frequently influence the individual's continued compliance with chemotherapy treatment protocols.

Accordingly, the compounds and methods of the invention may be used to reduce the amount of radiation, immunotherapy or chemotherapy required to control these conditions, and to overcome some of the limitations of these conventional therapies.

In many indications involving aberrant proliferation of Hematopoietic cells, there are two main treatment phases: remission induction and post-remission treatment. Post-remission treatment may be referred to as consolidation therapy. Less frequently, a third phase of treatment involving long-term, low-dose chemotherapy (maintenance therapy). Although maintenance therapy may reduce the likelihood of relapses, the general consensus is that this benefit is outweighed by the increased risk of treatment-related mortality when extended maintenance treatment is given.

Remission induction is achieved in most patients using two or more drugs in combination to clear all detectable cancerous cells from the blood and/or bone marrow. Remission induction is essentially standard for all patients except those with acute promyelocytic leukemia (APL), a subtype of the cancer acute myeloid leukemia (AML). Remission induction normally involves administration of the drug cytarabine, optionally in combination with an anthracycline (including but not limited to daunorubicin, mitoxantrone, or idarubicin). Sometimes a third drug, such as etoposide or thioguanine, is also administered. The intensity of treatment typically causes severe bone marrow suppression. Myeloid colony-stimulating factors (G-CSF and GM-CSF) can be administered to induce myeloid progenitor cell production and shorten the period of granulocytopenia following induction therapy. For acute promyelocytic leukemia, (M3 stage) tretinoin (all-trans-retinoic acid, ATRA) can be used to induce terminal differentiation of the leukemic cells (i.e., to induce the proliferating, immature cells to differentiate into non-proliferating, specialized, mature cells).

The disappearance of detectable cancerous cells from the blood and bone marrow does not necessarily mean that all malignant cells in the body have been killed. Thus, additional treatment with the same, or similar drugs as used in remission induction at the same, or lower doses are often administered soon after completion of the remission induction phase. In some treatment protocols, consolidation therapy is intensified by using cytarabine.

In one aspect, the method of the invention can be employed to treat subjects therapeutically or prophylactically who have or can be subject to an inflammatory disorder. One aspect of the present invention derives from the involvement of PI3Kδ in mediating aspects of the inflammatory process. Without intending to be bound by any theory, it is theorized that, because inflammation involves processes that are typically mediated by leukocyte (*e.g*., neutrophil, lymphocyte, etc.) activation and chemotactic transmigration, and because PI3Kδ can mediate such phenomena, antagonists of PI3Kδ can be used to suppress injury associated with inflammation.

The therapeutic methods of the present invention include methods for the treatment of disorders associated with inflammatory cell activation. "Inflammatory cell activation" refers to the induction by a stimulus (including, but not limited to, cytokines, antigens or auto-antibodies) of a proliferative cellular response, the production of soluble mediators (including but not limited to cytokines, oxygen radicals, enzymes, prostanoids, or vasoactive amines), or cell surface expression of new or increased numbers of mediators (including, but not limited to, major histocompatibility antigens or cell adhesion molecules) in inflammatory cells (including but not limited to monocytes, macrophages, T lymphocytes, B lymphocytes, granulocytes (*i.e*., polymorphonuclear leukocytes such as neutrophils, basophils, and eosinophils), mast cells, dendritic cells, Langerhans cells, and endothelial cells). It will be appreciated by persons skilled in the art that the activation of one or a combination of these phenotypes in these cells can contribute to the initiation, perpetuation, or exacerbation of an inflammatory disorder.

The compounds of the invention have been found to inhibit superoxide release by neutrophils. Superoxide is released by neutrophils in response to any of a variety of stimuli, including signals of infection, as a mechanism of cell killing. For example, superoxide release is known to be induced by tumor necrosis factor alpha (TNFα), which is released by macrophages, mast cells, and lymphocytes upon contact with bacterial cell wall components such as lipopolysaccharide (LPS). TNFα is an extraordinarily potent and promiscuous activator of inflammatory processes, being involved in activation of neutrophils and various other cell types, induction of leukocyte/endothelial cell adhesion, pyrexia, enhanced MHC Class I production, and stimulation of angiogenesis. Alternatively, superoxide release can be stimulated by formyl-Met-Leu-Phe (fMLP) or other peptides blocked at the N-terminus by formylated methionine. Such peptides are not normally found in eukaryotes, but are fundamentally characteristic of bacteria, and signal the presence of bacteria to the immune system. Leukocytes expressing the fMLP receptor, *e.g*., neutrophils and macrophages, are stimulated to migrate up gradients of these peptides (*i.e*., chemotaxis) toward loci of infection, As demonstrated herein, the compounds of the invention inhibit stimulated superoxide release by neutrophils in response to either TNFα or fMLP. Other functions of neutrophils, including stimulated exocytosis and directed chemotactic migration, also have been shown to be inhibited by the PI3Kδ inhibitors of the invention. Accordingly, the compounds of the invention can be expected to be useful in treating disorders, such as inflammatory disorders, that are mediated by any or all of these neutrophil functions.

The present invention enables methods of treating such diseases as arthritic diseases, including rheumatoid arthritis, monoarticular arthritis, osteoarthritis, gouty arthritis, spondylitis; Behçet disease; sepsis, septic shock, endotoxic shock, gram negative sepsis, gram positive sepsis, and toxic shock syndrome; multiple organ injury syndrome secondary to septicemia, trauma, or hemorrhage; ophthalmic disorders such as allergic conjunctivitis, vernal conjunctivitis, uveitis, and thyroid-associated ophthalmopathy; eosinophilic granuloma; pulmonary or respiratory disorders such as asthma, chronic bronchitis, allergic rhinitis, ARDS, chronic pulmonary inflammatory disease (e.g., chronic obstructive pulmonary disease), silicosis, pulmonary sarcoidosis, pleurisy, alveolitis, vasculitis, emphysema, pneumonia, bronchiectasis, and pulmonary oxygen toxicity; reperfusion injury of the myocardium, brain, or extremities; fibrosis such as cystic fibrosis; keloid formation or scar tissue formation; atherosclerosis; autoimmune diseases, such as multiple sclerosis, systemic lupus erythematosus (SLE), autoimmune thyroiditis, some forms of diabetes, and Raynaud's syndrome; and transplant rejection disorders such as GVHD and allograft rejection; chronic glomerulonephritis; inflammatory bowel diseases such as chronic inflammatory bowel disease (CIBD), Crohn's disease, ulcerative colitis, and necrotizing enterocolitis; inflammatory dermatoses such as contact dermatitis, atopic dermatitis, psoriasis, or urticaria; fever and myalgias due to infection; central or peripheral nervous system inflammatory disorders such as meningitis, encephalitis, and brain or spinal cord injury due to minor trauma; Sjogren's syndrome; diseases involving leukocyte diapedesis; alcoholic hepatitis; bacterial pneumonia; antigen-antibody complex mediated diseases; hypovolemic shock; Type I diabetes mellitus; acute and delayed hypersensitivity; disease states due to leukocyte dyscrasia and metastasis; thermal injury; granulocyte transfusion-associated syndromes; and cytokine-induced toxicity.

The method can have utility in treating subjects who are or can be subject to reperfusion injury, *i.e*., injury resulting from situations in which a tissue or organ experiences a period of ischemia followed by reperfusion. The term "ischemia" refers to localized tissue anemia due to obstruction of the inflow of arterial blood. Transient ischemia followed by reperfusion characteristically results in neutrophil activation and transmigration through the endothelium of the blood vessels in the affected area, Accumulation of activated neutrophils in turn results in generation of reactive oxygen metabolites, which damage components of the involved tissue or organ. This phenomenon of "reperfusion injury" is commonly associated with conditions such as vascular stroke (including global and focal ischemia), hemorrhagic shock, myocardial ischemia or infarction, organ transplantation, and cerebral vasospasm. To illustrate, reperfusion injury occurs at the termination of cardiac bypass procedures or during cardiac arrest when the heart, once prevented from receiving blood, begins to reperfuse. It is expected that inhibition of PI3K8 activity will result in reduced amounts of reperfusion injury in such situations.

With respect to the nervous system, global ischemia occurs when blood flow to the entire brain ceases for a period. Global ischemia can result from cardiac arrest. Focal ischemia occurs when a portion of the brain is deprived of its normal blood supply. Focal ischemia can result from thromboembolytic occlusion of a cerebral vessel, traumatic head injury, edema, or brain tumor. Even if transient, both global and focal ischemia can cause widespread neuronal damage. Although nerve tissue damage occurs over hours or even days following the onset of ischemia, some permanent nerve tissue damage can develop in the initial minutes following the cessation of blood flow to the brain.

Ischemia also can occur in the heart in myocardial infarction and other cardiovascular disorders in which the coronary arteries have been obstructed as a result of atherosclerosis, thrombus, or spasm. Accordingly, the invention is also useful for treating cardiac tissue damage, particularly damage resulting from cardiac ischemia or damage caused by reperfusion injury in mammals.

In another aspect, selective inhibitors of PI3Kδ activity, such as the compounds of the invention, can be employed in methods of treating diseases of bone, especially diseases in which osteoclast function is abnormal or undesirable. Selective inhibitors of PI3Kδ have been shown to inhibit osteoclast function *in vitro.* See, *e.g.*, U.S. Patent No. 6,800,620. Accordingly, the PI3Kδ selective inhibitors of the invention can be of value in treating osteoporosis, Paget's disease, and related bone resorption disorders where a reduction in osteoclast function is desired.

In a further aspect, the invention includes methods of using selective PI3Kδ inhibitory compounds to inhibit the growth or proliferation of cancer cells of hematopoietic origin, particularly cancer cells of lymphoid origin, and more particularly cancer cells related to or derived from B lymphocytes or B lymphocyte progenitors. Cancers amenable to treatment using the method of the invention include, without limitation, lymphomas, *e.g*., malignant neoplasms of lymphoid and reticuloendothelial tissues, such as Burkitt's lymphoma, Hodgkin's lymphoma, non-Hodgkin's lymphomas, lymphocytic lymphomas and the like; multiple myelomas; as well as leukemias such as lymphocytic leukemias, chronic myeloid (myelogenous) leukemias, and the like. In a preferred embodiment, PI3Kδ inhibitory compounds can be used to inhibit or control the growth or proliferation of chronic myeloid (myelogenous) leukemia cells.

In another aspect, the invention includes a method for suppressing a function of basophils and/or mast cells, and thereby enabling treatment of diseases or disorders characterized by excessive or undesirable basophil and/or mast cell activity. According to the method, a compound of the invention can be used to selectively inhibit the expression or activity of phosphatidylinositol 3-kinase delta (PI3Kδ) in the basophils and/or mast cells. Preferably, the method employs a PI3Kδ inhibitor of formula (1) or (2) in an amount sufficient to inhibit stimulated histamine release by the basophils and/or mast cells. Accordingly, the use of such PI3Kδ selective inhibitors can be of value in treating diseases characterized by histamine release, *i.e*., allergic disorders, including disorders such as chronic obstructive pulmonary disease (COPD), asthma, ARDS, emphysema, and related disorders.

The methods in accordance with the invention may include administering a PI3Kδ selective inhibitor with one or more other agents that either enhance the activity of the inhibitor or compliment its activity or use in treatment. Such additional factors and/or agents may produce an augmented or even synergistic effect when administered with a PI3Kδ selective inhibitor, or minimize side effects.

In one embodiment, the methods of the invention further comprise administering a mammalian target of rapamycin (mTOR) inhibitor. In one aspect of this embodiment, the mTOR inhibitor is rapamycin. Other mTOR inhibitors that may be used include FK506, cyclosporine A (CsA), and everolimus.

In one embodiment, the methods of the invention may include administering formulations comprising a selective PI3Kδ inhibitor of the invention with a particular cytokine, lymphokine, other hematopoietic factor, thrombolytic or anta-thrombotic factor, or anti-inflammatory agent before, during, or after administration of the PI3Kδ selective inhibitor. One of ordinary skill can easily determine if a particular cytokine, lymphokine, hematopoietic factor, thrombolytic or anti-thrombotic factor, and/or anti-inflammatory agent enhances or complements the activity or use of the PI3Kδ selective inhibitors in treatment.

More specifically, and without limitation, the methods of the invention may comprise administering a PI3Kδ selective inhibitor with one or more of TNF, IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, IFN, G-CSF, Meg-CSF, GM-CSF, thrombopoietin, stem cell factor, and erythropoietin. Compositions in accordance with the invention may also include other known angiopoietins such as Ang-2, Ang-4, and Ang-Y, growth factors such as bone morphogenic protein-1, bone morphogenic protein-2, bone morphogenic protein-3, bone morphogenic protein-4, bone morphogenic protein-5, bone morphogenic protein-6, bone morphogenic protein-7, bone morphogenic protein-8, bone morphogenic protein-9, bone morphogenic protein-10, bone morphogenic protein-11, bone morphogenic protein-12, bone morphogenic protein-13, bone morphogenic protein-14, bone morphogenic protein-15, bone morphogenic protein receptor IA, bone morphogenic protein receptor IB, brain derived neurotrophic factor, ciliary neutrophic factor, ciliary neutrophic factor receptor α, cytokine-induced neutrophil chemotactic factor 1, cytokine-induced neutrophil chemotactic factor 2α, cytokine-induced neutrophil chemotactic factor 2 β, β endothelial cell growth factor, endothelin 1, epidermal growth factor, epithelialderived neutrophil attractant, fibroblast growth factor 4, fibroblast growth factor 5, fibroblast growth factor 6, fibroblast growth factor 7, fibroblast growth factor 8, fibroblast growth factor 8b, fibroblast growth factor 8c, fibroblast growth factor 9, fibroblast growth factor 10, fibroblast growth factor acidic, fibroblast growth factor basic, glial cell line-derived neutrophic factor receptor al, glial cell line-derived neutrophic factor receptor α2, growth related protein, growth related protein α, growth related protein β, growth related protein γ, heparin binding epidermal growth factor, hepatocyte growth factor, hepatocyte growth factor receptor, insulin-like growth factor I, insulin-like growth factor receptor, insulin-like growth factor II, insulin-like growth factor binding protein, keratinocyte growth factor, leukemia inhibitory factor, leukemia inhibitory factor receptor α, nerve growth factor, nerve growth factor receptor, neurotrophin-3, neurotrophin-4, placenta growth factor, placenta growth factor 2, platelet derived endothelial cell growth factor, platelet derived growth factor, platelet derived growth factor A chain, platelet derived growth factor AA, platelet derived growth factor AB, platelet derived growth factor B chain, platelet derived growth factor BB, platelet derived growth factor receptor α, platelet derived growth factor receptor β, pre-B cell growth stimulating factor, stem cell factor, stem cell factor receptor, transforming growth factor α, transforming growth factor β, transforming growth factor β1, transforming growth factor β1.2, transforming growth factor β2, transforming growth factor β3, transforming growth factor β5, latent transforming growth factor β1, transforming growth factor β binding protein I, transforming growth factor β binding protein II, transforming growth factor β binding protein III, tumor necrosis factor receptor type I, tumor necrosis factor receptor type II, urokinase-type plasminogen activator receptor, and chimeric proteins and biologically or immunologically active fragments thereof.

Additionally, and without limitation, the methods of the invention may comprise administering a PI3Kδ selective inhibitor with one or more chemotherapeutic agents including but not limited to alkylating agents, intercalating agents, antimetabolites, natural products, biological response modifiers, miscellaneous agents, and hormones and antagonists. Alkylating agents for use in the inventive methods include but are not limited to nitrogen mustards such as mechlorethamine, cyclophosphamide, ifosfamide, melphalan and chlorambucil, nitrosoureas such as carmustine (BCNU), lomustine (CCNU) and semustine (methyl-CCNU), ethylenimine/methylmelamines such as triethylenemelamine (TEM), triethylene thiophosphoramide (thiotepa) and hexamethylmelamine (HMM, altretamine), alkyl sulfonates such as busulfan, and triazines such as dacarbazine (DTIC). Antimetabolites include but are not limited to folic acid analogs (including methotrexate, trimetrexate, and pemetrexed disodium), pyrimidine analogs (including 5-fluorouracil, fluorodeoxyuridine, gemcitabine, cytosine arabinoside (AraC, cytarabine), 5-azacytidine and 2,2-difluorodeoxycytidine), and purine analogs (including 6-mercaptopurine, 6-thioguanine, azathioprine, 2'-deoxycoformycin (pentostatin), erythrohydroxynonyladenine (EHNA), fludarabine phosphate and 2-chlorodeoxyadenosine (cladribine, 2-CdA)). Intercalating agents for use in the inventive methods include but are not limited to ethidium bromide and acridine. Natural products for use in the inventive methods include but are not limited to antimitotic drugs such as paclitaxel, docetaxel, vinca alkaloids (including vinblastine (VLB), vincristine, vindesine and vinorelbine), taxotere, estramustine and estramustine phosphate. Additional natural products for use in the inventive methods include epipodophyllotoxins such as etoposide and teniposide, antibiotics such as actinomycin D, daunomycin (rubidomycin), doxorubicin, mitoxantrone, idarubicin, bleomycins, plicamycin (mithramycin), mitomycin C, dactinomycin and actinomycin D, and enzymes such as L-asparaginase. Biological response modifiers for use in the inventive methods include but are not limited to interferon-alpha, IL-2, G-CSF and GM-CSF. Miscellaneous agents for use in the inventive methods include but are not limited to platinum coordination complexes such as cisplatin and carboplatin, anthracenediones such as mitoxantrone, substituted ureas such as hydroxyurea, methylhydrazine derivatives such as N-methylhydrazine (MIH) and procarbazine, and adrenocortical suppressants such as mitotane (o,p*-DDD) and aminoglutethimide. Hormones and antagonists for use in the inventive methods include but are not limited to adrenocorticosteroids/antagonists such as prednisone, dexamethasone and aminoglutethimide, progestins such as hydroxyprogesterone caproate, medroxyprogesterone acetate and megestrol acetate, estrogens such as diethylstilbestrol and ethinyl estradiol, antiestrogens such as tamoxifen, androgens such as testosterone propionate and fluoxymesterone, antiandrogens such as flutamide, gonadotropin-releasing hormone analogs and leuprolide, and non-steroidal antiandrogens such as flutamide.

In one aspect, the chemotherapeutic is a DNA-damaging chemotherapeutic. Specific types of DNA-damaging chemotherapeutic agents contemplated for use in the inventive methods include, *e.g*., alkylating agents and intercalating agents.

The methods of the invention can also further comprise administering a PI3Kδ selective inhibitor in combination with a photodynamic therapy protocol. Typically, a photosensitizer is administered orally, intravenously, or topically, and then activated by an external light source. Photosensitizers for use in the methods of the invention include but are not limited to psoralens, lutetium texaphyrin (LuTex), benzoporphyrin derivatives (BPD) such as Verteporfin and Photofrin porfimer sodium (PH), phthalocyanines and derivatives thereof. Lasers are typically used to activate the photosensitizer. Light-emitting diodes (LEDs) and florescent light sources can also be used, but these do result in longer treatment times.

Additionally, and without limitation, the methods of the invention may comprise administering a PI3Kδ selective inhibitor at least one anti-angiogenic agent including but not limited to plasminogen fragments such as angiostatin and endostatin; angiostatic steroids such as squalamine; matrix metalloproteinase inhibitors such as Bay-129566; anti-vascular endothelial growth factor (anti-VEGF) isoform antibodies; anti-VEGF receptor antibodies; inhibitors that target VEGF isoforms and their receptors; inhibitors of growth factor (*e.g*., VEGF, PDGF, FGF) receptor tyrosine kinase catalytic activity such as SU11248; inhibitors of FGF production such as interferon alpha; inhibitors of methionine aminopeptidase-2 such as TNP-470; copper reduction therapies such as tetrathiomolybdate; inhibitors of FGF-triggered angiogenesis such as thalidomide and analogues thereof; platelet factor 4; and thrombospondin.

Additionally, the methods of the invention can further comprise bone marrow transplantation (BMT) and/or peripheral blood stem cell transplantation (PBSCT) procedures. The transplants may alternatively be autologous transplants, syngeneic transplants, or allogeneic transplants.

Methods of the invention contemplate use of a PI3Kδ selective inhibitor compound having formula (I) or a pharmaceutically acceptable salt or solvate thereof: 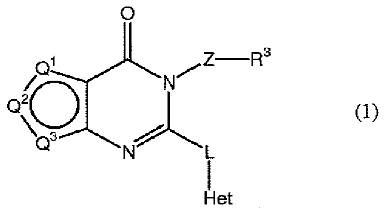 wherein Q¹, Q², Q³, Z, R³, L and Het are as defined above.

The inhibitors of the invention may be covalently or noncovalently associated with a carrier molecule including but not limited to a linear polymer (*e.g*., polyethylene glycol, polylysine, dextran, etc.), a branched-chain polymer (see U.S. Patent Nos. 4,289,872 and 5,229,490; PCT Publication No. WO 93/21259), a lipid, a cholesterol group (such as a steroid), or a carbohydrate or oligosaccharide. Specific examples of carriers for use in the pharmaceutical compositions of the invention include carbohydrate-based polymers such as trehalose, mannitol, xylitol, sucrose, lactose, sorbitol, dextrans such as cyclodextran, cellulose, and cellulose derivatives. Also, the use of liposomes, microcapsules or microspheres, inclusion complexes, or other types of carriers is contemplated.

Other carriers include one or more water soluble polymer attachments such as polyoxyethylene glycol, or polypropylene glycol as described U.S. Patent Nos. 4,640,835, 4,496,689, 4,301,144, 4,670,417, 4,791,192 and 4,179,337. Still other useful carrier polymers known in the art include monomethoxy-polyethylene glycol, poly-(N-vinyl pyrrolidone)-polyethylene glycol, propylene glycol homopolymers, a polypropylene oxide/ethylene oxide co-polymer, polyoxyethylated polyols (*e.g.*, glycerol) and polyvinyl alcohol, as well as mixtures of these polymers.

Methods include administration of an inhibitor to an individual in need, by itself, or in combination as described herein, and in each case optionally including one or more suitable diluents, fillers, salts, disintegrants, binders, lubricants, glidants, wetting agents, controlled release matrices, colorants/flavoring, carriers, excipients, buffers, stabilizers, solubilizers, other materials well known in the art and combinations thereof.

Any pharmaceutically acceptable (*i.e*., sterile and non-toxic) liquid, semisolid, or solid diluents that serve as pharmaceutical vehicles, excipients, or media may be used. Exemplary diluents include, but are not limited to, polyoxyethylene sorbitan monolaurate, magnesium stearate, calcium phosphate, mineral oil, cocoa butter, and oil of theobroma, methyl- and propylhydroxybenzoate, talc, alginates, carbohydrates, especially mannitol, α-lactose, anhydrous lactose, cellulose, sucrose, dextrose, sorbitol, modified dextrans, gum acacia, and starch. Some commercially available diluents are Fast-Flo^{®}, Emdex^{®}, STA-Rx 1500^{®}, Emcompress^{®} and Avicel^{®}, Such compositions may influence the physical state, stability, rate *of in vivo* release, and rate *of in vivo* clearance of the PI3Kδ inhibitor compounds (see, *e.g*., Remington's Pharmaceutical Sciences, 18th Ed. pp. 1435-1712 (1990), which is incorporated herein by reference).

Pharmaceutically acceptable fillers can include, for example, lactose, microcrystalline cellulose, dicalcium phosphate, tricalcium phosphate, calcium sulfate, dextrose, mannitol, and/or sucrose.

Inorganic salts including calcium triphosphate, magnesium carbonate, and sodium chloride may also be used as fillers in the pharmaceutical compositions. Amino acids may be used such as use in a buffer formulation of the pharmaceutical compositions.

Disintegrants may be included in solid dosage formulations of the inhibitors. Materials used as disintegrants include but are not limited to starch including the commercial disintegrant based on starch, Explotab^{®}. Sodium starch glycolate, Amberlite^{®}, sodium carboxymethylcellulose, ultramylopectin, sodium alginate, gelatin, orange peel, acid carboxymethylcellulose, natural sponge and bentonite may all be used as disintegrants in the pharmaceutical compositions. Other disintegrants include insoluble cationic exchange resins. Powdered gums including powdered gums such as agar, karaya or tragacanth may be used as disintegrants and as binders. Alginic acid and its sodium salt are also useful as disintegrants.

Binders may be used to hold the therapeutic agent together to form a hard tablet and include materials from natural products such as acacia, tragacanth, starch and gelatin. Others include methyl cellulose (MC), ethyl cellulose (EC) and carboxymethyl cellulose (CMC). Polyvinyl pyrrolidone (PVP) and hydroxypropylmethyl cellulose (HPMC) can both be used in alcoholic solutions to facilitate granulation of the therapeutic ingredient.

An antifrictional agent may be included in the formulation of the therapeutic ingredient to prevent sticking during the formulation process. Lubricants may be used as a layer between the therapeutic ingredient and the die wall, and these can include but are not limited to; stearic acid including its magnesium and calcium salts, polytetrafluoroethylene (PTFE), liquid paraffin, vegetable oils and waxes. Soluble lubricants may also be used such as sodium lauryl sulfate, magnesium lauryl sulfate, polyethylene glycol of various molecular weights, Carbowax^{®} 4000 and 6000.

Glidants that might improve the flow properties of the therapeutic ingredient during formulation and to aid rearrangement during compression might be added. Suitable glidants include starch, talc, pyrogenic silica and hydrated silicoaluminate.

To aid dissolution of the therapeutic into the aqueous environment, a surfactant might be added as a wetting agent. Natural or synthetic surfactants may be used. Surfactants may include anionic detergents such as sodium lauryl sulfate, dioctyl sodium sulfosuccinate, and dioctyl sodium sulfonate. Cationic detergents such as benzalkonium chloride and benzethonium chloride may be used. Nonionic detergents that can be used in the pharmaceutical formulations include lauromacrogol 400, polyoxyl 40 stearate, polyoxyethylene hydrogenated castor oil 10, 50 and 60, glycerol monostearate, polysorbate 40, 60, 65 and 80, sucrose fatty acid ester, methyl cellulose and carboxymethyl cellulose. These surfactants can be present in the pharmaceutical compositions of the invention either alone or as a mixture in different ratios.

Controlled release formulation may be desirable. The inhibitors of the invention can be incorporated into an inert matrix which permits release by either diffusion or leaching mechanisms, *e.g*., gums. Slowly degenerating matrices may also be incorporated into the pharmaceutical formulations, *e.g*., alginates, polysaccharides. Another form of controlled release is a method based on the Uros^{®} therapeutic system (Alza Corp.), *i.e*., the drug is enclosed in a semipermeable membrane which allows water to enter and push the inhibitor compound out through a single small opening due to osmotic effects. Some enteric coatings also have a delayed release effect.

Colorants and flavoring agents may also be included in the pharmaceutical compositions. For example, the inhibitors of the invention may be formulated (such as by liposome or microsphere encapsulation) and then further contained within an edible product, such as a beverage containing colorants and flavoring agents.

The therapeutic agent can also be given in a film coated tablet. Nonenteric materials for use in coating the pharmaceutical compositions include methyl cellulose, ethyl cellulose, hydroxyethyl cellulose, methylhydroxy-ethyl cellulose, hydroxypropyl cellulose, hydroxypropyl-methyl cellulose, sodium carboxy-methyl cellulose, povidone and polyethylene glycols. Enteric materials for use in coating the pharmaceutical compositions include esters of phthalic acid. A mix of materials might be used to provide the optimum film coating. Film coating manufacturing may be carried out in a pan coater, in a fluidized bed, or by compression coating.

The compositions can be administered in solid, semi-solid, liquid or gaseous form, or may be in dried powder, such as lyophilized form. The pharmaceutical compositions can be packaged in forms convenient for delivery, including, for example, capsules, sachets, cachets, gelatins, papers, tablets, capsules, suppositories, pellets, pills, troches, lozenges or other forms known in the art. The type of packaging will generally depend on the desired route of administration. Implantable sustained release formulations are also contemplated, as are transdermal formulations.

In the methods according to the invention, the inhibitor compounds may be administered by various routes. For example, pharmaceutical compositions may be for injection, or for oral, nasal, transdermal or other forms of administration, including, *e.g.*, by intravenous, intradermal, intramuscular, intramammary, intraperitoneal, intrathecal, intraocular, retrobulbar, intrapulmonary (*e.g*., aerosolized drugs) or subcutaneous injection (including depot administration for long term release, *e.g*., embedded under the splenic capsule, brain, or in the cornea); by sublingual, anal, vaginal, or by surgical implantation, *e.g*., embedded under the splenic capsule, brain, or in the cornea. The treatment may consist of a single dose or a plurality of doses over a period of time. In general, the methods of the invention involve administering effective amounts of an inhibitor of the invention together with pharmaceutically acceptable diluents, preservatives, solubilizers, emulsifiers, adjuvants and/or carriers, as described above.

In one aspect, the invention provides methods for oral administration of a pharmaceutical composition of the invention. Oral solid dosage forms are described generally in Remington's Pharmaceutical Sciences, *supra* at chapter 89. Solid dosage forms include tablets, capsules, pills, troches or lozenges, and cachets or pellets. Also, liposomal or proteinoid encapsulation may be used to formulate the compositions (as, for example, proteinoid microspheres reported in U.S. Patent No. 4,925,673). Liposomal encapsulation may include liposomes that are derivatized with various polymers (*e.g*., U.S. Patent No. 5,013,556). In general, the formulation will include a compound of the invention and inert ingredients which protect against degradation in the stomach and which permit release of the biologically active material in the intestine.

The inhibitors can be included in the formulation as fine multiparticulates in the form of granules or pellets of particle size about 1 mm. The formulation of the material for capsule administration could also be as a powder, lightly compressed plugs or even as tablets. The capsules could be prepared by compression.

Also contemplated herein is pulmonary delivery of the PI3Kδ inhibitors in accordance with the invention. According to this aspect of the invention, the inhibitor is delivered to the lungs of a mammal by inhalation of a suitable composition, and the PI3Kδ inhibitor traverses across the lung epithelial lining to the blood stream.

Contemplated for use in the practice of this invention are a wide range of mechanical devices designed for pulmonary delivery of therapeutic products, including but not limited to nebulizers, metered dose inhalers, and powder inhalers, all of which are familiar to those skilled in the art. Some specific examples of commercially available devices suitable for the practice of this invention are the UltraVent™ nebulizer, manufactured by Mallinckrodt, Inc., St. Louis, Mo,; the Acorn II^{®} nebulizer, manufactured by Marquest Medical Products, Englewood, Colorado; the Ventolin^{®} metered dose inhaler, manufactured by Glaxo Inc., Research Triangle Park, North Carolina; and the Spinhaler^{®} powder inhaler, manufactured by Fisons Corp., Bedford, Mass.

All such devices require the use of formulation suitable for the dispensing of the inventive compound. Typically, each formulation is specific to the type of device employed and may involve the use of an appropriate propellant material, in addition to diluents, adjuvants and/or carriers useful in therapy.

When used in pulmonary administration methods, the inhibitors of the invention are most advantageously prepared in particulate form with an average particle size of less than 10 µm (or microns), for example, 0.5 to 5 µm, for most effective delivery to the distal lung.

Formulations suitable for use with a nebulizer, either jet or ultrasonic, will typically comprise the inventive compound dissolved in water at a concentration range of about 0.1 to 100 mg of inhibitor per mL of solution, 1 to 50 mg of inhibitor per mL of solution, or 5 to 25 mg of inhibitor per mL of solution. The formulation may also include a buffer. The nebulizer formulation may also contain a surfactant, to reduce or prevent surface induced aggregation of the inhibitor caused by atomization of the solution in forming the aerosol.

Formulations for use with a metered-dose inhaler device will generally comprise a finely divided powder containing the inventive inhibitors suspended in a propellant with the aid of a surfactant. The propellant may be any conventional material employed for this purpose, such as a chlorofluorocarbon, a hydrochlorofluorocarbon, a hydrofluorocarbon, or a hydrocarbon, including trichlorofluoromethane, dichlorodifluoromethane, dichlorotetrafluoroethanol, and 1,1,1,2-tetrafluoroethane, or combinations thereof. Suitable surfactants include sorbitan trioleate and soya lecithin. Oleic acid may also be useful as a surfactant.

Formulations for dispensing from a powder inhaler device will comprise a finely divided dry powder containing the inventive compound and may also include a bulking agent or diluent such as lactose, sorbitol, sucrose, mannitol, trehalose, or xylitol in amounts which facilitate dispersal of the powder from the device, *e.g.*, 50 to 90% by weight of the formulation.

Nasal delivery of the inventive compound is also contemplated. Nasal delivery allows the passage of the inhibitor to the blood stream directly after administering the therapeutic product to the nose, without the necessity for deposition of the product in the lung. Formulations for nasal delivery may include dextran or cyclodextran. Delivery via transport across other mucous membranes is also contemplated.

Toxicity and therapeutic efficacy of the PI3Kδ selective compounds can be determined by standard pharmaceutical procedures in cell cultures or experimental animals, *e.g.*, for determining the LD50 (the dose lethal to 50% of the population) and the ED50 (the dose therapeutically effective in 50% of the population). Additionally, this information can be determined in cell cultures or experimental animals additionally treated with other therapies including but not limited to radiation, chemotherapeutic agents, photodynamic therapies, radiofrequency ablation, anti-angiogenic agents, and combinations thereof.

In practice of the methods of the invention, the pharmaceutical compositions are generally provided in doses ranging from 1 pg compound/kg body weight to 1000 mg/kg, 0.01 mg/kg to 100 mg/kg, 0.1 mg/kg to 20 mg/kg, given in daily doses or in equivalent doses at longer or shorter intervals, *e.g*., every other day, twice weekly, weekly, or twice or three times daily. The inhibitor compositions may be administered by an initial bolus followed by a continuous infusion to maintain therapeutic circulating levels of drug product. Those of ordinary skill in the art will readily optimize effective dosages and administration regimens as determined by good medical practice and the clinical condition of the individual to be treated. The frequency of dosing will depend on the pharmacokinetic parameters of the agents and the route of administration.

The optimal pharmaceutical formulation will be determined by one skilled in the art depending upon the route of administration and desired dosage (see, for example, Remington's Pharmaceutical Sciences, latest ed., the disclosure of which is hereby incorporated by reference). Such formulations may influence the physical state, stability, rate of in vivo release, and rate of in *vivo* clearance of the administered agents. Depending on the route of administration, a suitable dose may be calculated according to body weight, body surface area or organ size. Further refinement of the calculations necessary to determine the appropriate dosage for treatment involving each of the above mentioned formulations is routinely made by those of ordinary skill in the art without undue experimentation, especially in light of the dosage information and assays disclosed herein, as well as the pharmacokinetic data observed in human clinical trials. Appropriate dosages may be ascertained by using established assays for determining blood level dosages in conjunction with an appropriate physician considering various factors which modify the action of drugs, *e.g*., the drug's specific activity, the severity of the indication, and the responsiveness of the individual, the age, condition, body weight, sex and diet of the individual, the time of administration and other clinical factors. As studies are conducted, further information will emerge regarding the appropriate dosage levels and duration of treatment for various indications involving aberrant proliferation of Hematopoietic cells.

### EXAMPLES

The following examples are provided merely to illustrate the invention, and are not intended to limit the scope thereof.

### Example 1

### Preparation of IC491691: 6-Bromo-3-phenyl-2-(9H-purin-6-ylsulfanylmethyl)-3H thieno[2,3-d]pyrimidin-4-one

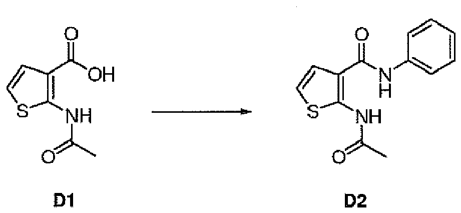

**D2: 2-acetylamino-thiophene-3-carboxylic acid phenylamide. A** solution of commercially available 2-acetylamino-thiophene-3-carboxylic acid (200 mg, 1.08 mmol), *O*-(7-azabenzotriazol-1-yl)-*N,N,N',N*'-tetramethyluronium hexafluorophosphate (HATU) (426 mg, 1.12 mmol), DIEA (376 µL, 2.16 mmol), and aniline (148 µL, 1.62 mmol) in DMF (2 mL) was stirred at room temperature for 16 h. The reaction mixture was then treated with H₂O (7 mL), stirred for 5 min., and a tan precipitate formed. The precipitate was collected by filtering through filter paper. The precipitate was then dissolved in ethyl acetate (10 mL) and washed with 3N HCl (2 x 15 mL). The organic layer was dried over magnesium sulfate, filtered, and concentrated by rotary evaporation to afford the product without further purification. LC/MS (AP-ESI, CH₃CO₂H 0.05%) *m*/*z* 261 (MH+).

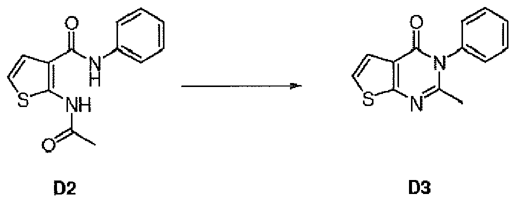

**D3: 2-methyl-3-phenyl-3*H*-thieno[2,34]pyrimidin-4-one. A solution** of 2-acetylamino-thiophene-3-carboxylic acid phenylamide (115 mgs, 0.442 mmol) in phosphorous oxychloride (5 mL) was heated to 125°C in a sealed tube for 48 h. The reaction mixture was allowed to cool to room temperature, and then concentrated to afford a residue. The residue was then dissolved in ethyl acetate (5 mL), and washed with saturated aqueous sodium bicarbonate solution (10 mL). The organic extract was dried over magnesium sulfate, filtered, and concentrated by rotary evaporation to afford the product without further purification as a green viscous residue. LC/MS (AP-ESI, CH₃CO₂H 0.05%) *m*/*z* 243 (MH+).

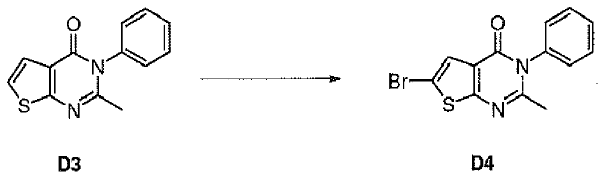

**D4: 6-Bromo-2-methyl-3-phenyl-3*H*-thieno[2,3,*d*]pyrimidin-4-one.** Following the general procedure described for IC-A7. A solution of 2-methyl-3-phenyl-3*H*-thieno[2,3,*d*]pyrimidin-4-one (118 mgs, 0.487 mmol) in acetic acid (3 mL) was treated with potassium acetate (72 mgs, 0.731 mmol), followed by addition of bromine (38 µL, 0.731 mmol). Purification by silica gel chromatography (9:1 hexanes/ethyl acetate) afforded product. LC/MS (AP-ESI, CH₃CO₂H 0.05%) *mlz* 323 (MH+).

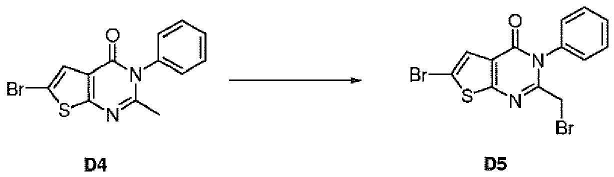

**D5: 6-Bromo-2-bromomethyl-3-phenyl-3*H*-thieno[2,3-d]pyrimidin-4-one.** A solution of 6-bromo-2-methyl-3-phenyl-3*H*-thieno[2,3,*d*]pyrimidin-4-one (90 mg, 0.280 mmol) in carbon tetrachloride (3 mL) was treated with N-bromosuccinimide (84 mg, 0.476 mmol), followed by addition of benzoyl peroxide (68 mg, 0.280). The resulting mixture was heated to reflux for 7.5 h, then cooled to room temperature, and filtered through silica gel. The silica gel plug was flushed with ethyl acetate (20 mL), and the combined filtrates were concentrated by rotary evaporation to afford the crude product (80 mg). Purification by HPLC (C-18 Vydac column 5.0 x 25 cm, 10-20% CH₃CN/H₂O containing 0.05% CHCO₂H), and subsequent lyophilizing afforded purified product as a white solid, LC/MS (AP-ESI, CH₃CO₂H0.05%) m/z 401 (MH+).

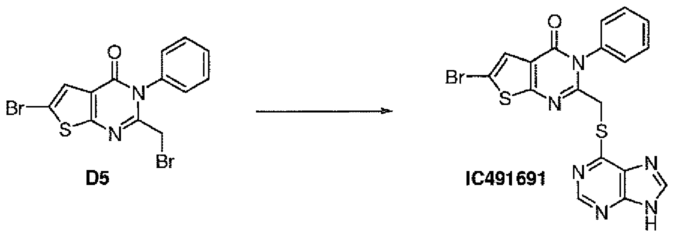

**IC491691: 6-Bromo-3-phenyl-2-(9*H*-purin-6-ylsuffanylmethyl)-3*H-*thieno[2,3-*d*]pyrimidin-4-one.** 6-bromo-2-bromomethyl-3-phenyl-3*H*-thieno[2,3-*d*]yrimidin-4-one (18 mg, 0.045 mmol) in DMF (500 µL) was treated with 6-mercaptopurine monohydrate (8 mg, 0.045 mmol) followed by the addition of potassium carbonate (8 mg, 0.045 mmol). The resulting mixture was stirred at room temperature for 16 h, and then quenched by adding saturated, aqueous sodium chloride solution (5 mL), which gave a white precipitate. After filtering, the product was obtained as a white solid. Crude reaction mixture was purified via HPLC (C-18 Luna column 1x18 mm, 10-20% CH₃CN/H₂O̅ containing 0.05% CF₃CO₂H) product was obtained as a fluffy white solid after lyophilizing: ¹H NMR (400 MHz, DMSO-d₆) δ 8.52 (s, 1H), 8.44 (s, 1H), 7.59 (s, 1H), 7.48 (m, 5H), 4.37 (s, 2H); LC/MS (AP-ESI, CHCO₂H 0.05%) *m*/*z* (MH+) 473.

### Examples 2

### Preparation of IC491693: 2-(6-Amino-purin-9-ylmethyl)-6-bromo-3-phenyl-3H-thieno-[2,3-d]pyrimidin-4-one

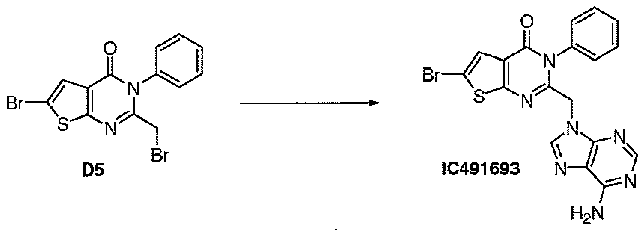

**IC491693: 6-Bromo-3-phenyl-2-(9*H*-purin-6-ylsulfanylmethyl)-3*H-*thieno[2,3-*d*]pyrimidin-4-one.** Following the general procedure described for IC491691. A stirring solution of 6-bromo-2-bromomethyl-3-phenyl-3*H*-thieno[2,3-d]pyrimidin-4-one (18 mg, 0.045 mmol) in DMF (500 µL) was treated with adenine (6.5 mg, 0.048 mmol) followed by the addition of potassium carbonate (6.5 mg, 0.047 mmol). Crude reaction mixture was purified via HPLC (C-18 Luna column 1x18 mm, 10-20% CH₃CN/H₂O containing 0.05% CF₃CO₂H) product was obtained as a fluffy white solid after lyophilizing: ¹H NMR (400 MHz, DMSO-D₆) δ 8.20 (s, 1H), 8.15 (s, 1H), 7.58 (m, 6H), 5.05 (s, 2H); LC/MS (AP-ESI, CH₃CO₂H 0.05%) *m*/*z* 456 (MH+).

### Example 3

### Preparation of IC491791: 2-[1-(4-Amino-benzoimidazol-1-yl)-ethyl]-3-phenyl-3H-thieno[3,2-d]pyrimidin-4-one

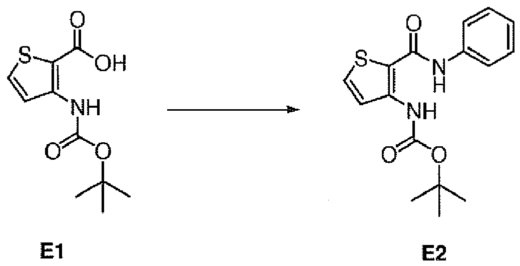

**E2: (2-Phenylcarbamoyl-thiophen-3-yl)-carbamic acid tert-butyl ester.** To a solution of 3-tert-butoxycarbonylamino-thiophene-2-carboxylic acid (1.5 g, 6.17 mmol) in DMF (15 mL) was added diisopropylethylamine (DIEA) (2.14 mL, 12.3 mmol), aniline (0.844 mL, 9.26 mmol), and *O*-(7-azabenzotriazol-1-yl)-*N,N,N',N*'-tetramethyluronium hexafluorophosphate (HATU) (2.68 g., 7.04 mmol) was stirred at room temperature for 3 h. The reaction mixture was then treated with H₂O (30 mL). Added ethyl acetate (75 mL), followed by a solution of saturated aqueous sodium carbonate, and washed the organic layer. The organic layer was then washed again sequentially with H₂O (60 mL), a solution of saturated aqueous sodium carbonate (1 x 60 mL), H₂O (60 mL), 1*N* HCl (1 x 60 mL), and H₂O (60 mL), The organic layer was then dried over magnesium sulfate, filtered and concentrated to afford the product as a golden oil. LC/MS (AP-ESI, CH₃CO₂H 0.05%) *m*/*z* 319 (MH+).

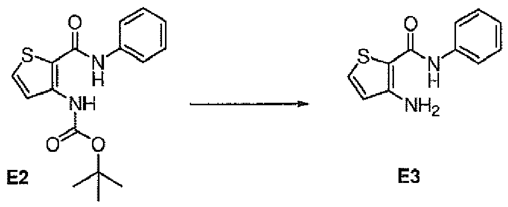

**E3: 3-Amino-thiophene-2-carboxylic acid phenylamide.** A solution of (2-Phenylcarbamoyl-thiophen-3-yl)-carbamic acid tert-butyl ester in trifluoroacetic acid/dichloromethane (1:1) was stirred at room temperature for 3 h. The reaction mixture was concentrated to a residue. The residue was dissolved in dichloromethane (50 mL), and then washed with a solution of saturated sodium carbonate (1 x 50 mL). The organic layer was dried over magnesium sulfate, filtered, and concentrated to afford the product without further purification. LC/MS (AP-ESI, CH₃CO₂H 0.05%) *m*/*z* 219 (MH+).

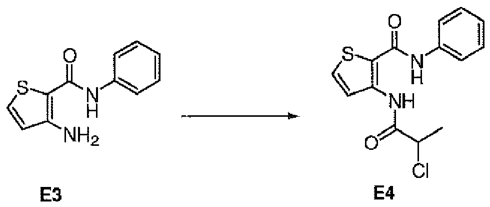

**E4: 3-(2-Chloro-propionylamino)-thiophene-2-carboxylic acid phenylamide.** To a solution of 3-Amino-thiophene-2-carboxylic acid phenylamide (1.25 g, 5.73 mmol) at 0°C was added 2-chloropropionyl chloride (0.477 mL, 4.81 mmol), and stirred for 1 h. Reaction mixture was then treated with H₂O (20 mL). The aqueous layer was extracted with dichloromethane (1 x 25 mL). The organic layers were combined and dried over magnesium sulfate, filtered, and concentrated to afford the product as a pale-white solid without further purification. LC/MS (AP-ESI, CH₃CO₂H 0.05%) *m*/*z* 309 (MH+).

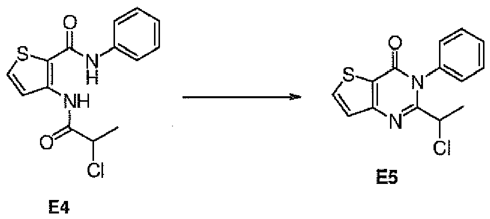

**E5: 2-(1-Chloro-ethyl)-3-phenyl-3H-thieno[3,2-d]pyrimidin-4-one.** A solution of 3-(2-Chloro-propionylamino)-thiophene-2-carboxylic acid phenylamide (600 mgs, 1.94 mmol) in phosphorous oxychloride (15 mL) was heated to 125°C in a sealed tube for 48 h. The reaction mixture was allowed to cool to room temperature, and then concentrated to afford a residue. The residue was then dissolved in ethyl acetate (25 mL), and washed with H₂O (2 x 25 mL). The organic extract was dried over magnesium sulfate, filtered, and concentrated by rotary evaporation to afford the product without further purification as a green viscous residue. LC/MS (AP-ESI, CH₃CO₂H 0.05%) *m*/*z* 291 (MH+).

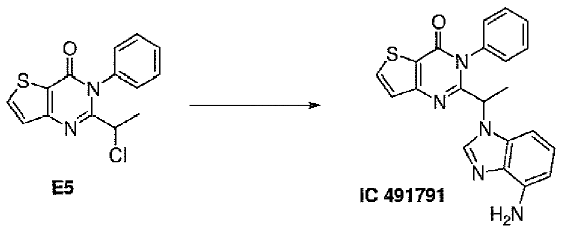

**IC491791: 2-[1-(4-Amino-benzoimidazol-1-yl)-ethyl]-3-phenyl-3H-thieno[3,2-d]pyrimidin-4-one.** To a solution of 2-(1-Amino-ethyl)-3-phenyl-3H-thieno[3,2-d]pyrimidin-4-one (47 mg, 0.162 mmol) in DMF was added adenine (26 mg, 0.194), followed by the addition of potassium carbonate (22 mg, 0.162 mmol). The resulting mixture was then heated to 125°C in an oil bath for 5 min. The reaction mixture was then cooled to room temperature, then treated with H₂O (10 mL) to afford a precipitate. The precipitate was collected and dried in a vacuum oven to afford the crude product. Purification by HPLC (C-18 Luna column 250 x 21.20 mm, 10-20% CH₃CN/H₂O containing 0.05% CF₃CO₂H) product was obtained as a fluffy white solid after lyophilizing: ¹H NMR (400 MHz, DMSO-d₆) δ 8.37 (s, 1H), 8.25 (d, *J* = 5.2 Hz, 1H), 8.16 (s, 1H), 7.67 (d, *J* = 8.0 Hz, 1H), 7.58 (m, 1H), 7.44 (m, 2H), 7.36 (m, 1H), 7.17 (d, *J* = 8.0 Hz, 1H), 5.49 (q, *J* = 6.8 Hz, 1H), 1.75 (d, J = 6,4 Hz, 3H). LC/MS (AP-ESI, CH₃CO₂H 0.05%) *m*/*z* 390 (MH+),

Note: Intermediate E5 can also be converted to a free amine, E6, by the following method; E6 is useful for the preparation of additional compounds within the scope of the invention. See, e.g., Example 6.

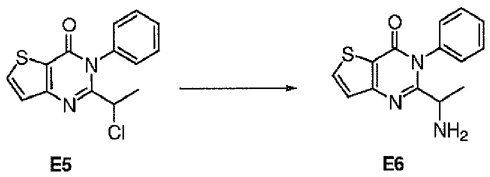

**E6: 2-(1-Amino-ethyl)-3-phenyl-3H-thieno[3,2-d]pyrimidin-4-one.** A solution of 2-(1-chloro-ethyl)-3-phenyl-3H-thieno[3,2-d]pyrimidin-4-one (564 mgs, 1.94 mmol) in 7*N* NH₃/MeOH was heated to 85°C for 48 h in a sealed tube, then cooled to room temperature, and concentrated to afford the product. LC/MS (AP-ESI, CH₃CO₂H 0.05%) *m*/*z* 272 (MH+).

### Example 4

### Preparation of IC491793: 3-Phenyl-2-[1-(9H-purin-6-ylsulfanyl)-ethyl]-3H-thieno[3,2-d]pyrimidin-4-one

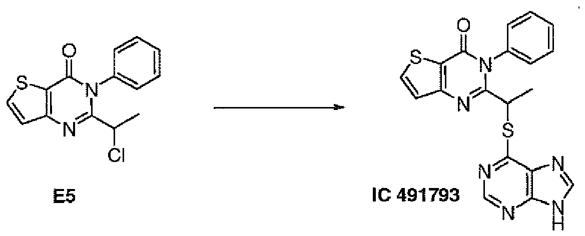

**IC491793**: 3-Phenyl-2-[1-(9H-purin-6-ylsulfanyl)-ethyl]-3H-thieno[3,2-d]pyrimidin-4-one. To a solution of 2-(1-Chloro-ethyl)-3-phenyl-3H-thieno[3,2-d]pyrimidin-4-one (47 mg, 0.162 mmol) (E5, preparation shown above) in DMF was added 6-mercaptopurine (33 mg, 0.194), followed by the addition of potassium carbonate (22 mg, 0.162 mmol). The resulting mixture was allowed to stir at room temperature for 18 h. The reaction mixture was then treated with H₂O (3 mL), and the aqueous layer was extracted ethyl acetate (3 x 15 mL). The organic extracts were then dried over magnesium sulfate, filtered, and concentrated to afford the crude product. The crude material was then purified by HPLC (C-18 Luna column 250 x 21.20 mm, 10-20% CH₃CN/H₂O containing 0.05% CF₃CO₂H) product was obtained after lyophilizing: ¹H NMR (400 MHz, DMSO-d₆) δ 8.40 (d, *J* = 8.0 Hz, 2H), 8.26 (d, *J* = 5.2 Hz, 1H), 7.54 (m, 3H), 7.34 (m, 2H), 7.13 (t, *J* = 7.6 Hz, 1H), 5.11 (q, *J* = 7.2 Hz, 1H), 1.68 (d, *J* = 6.8 Hz, 3H). LC/MS (AP-ESI, CH₃CO₂H 0.05%) *m*/*z* 407 (MH+).

### Example 5

### Preparation of IC491835: 3-Phenyl-2-[1-(9H-purin-6-ylamino)-ethyl]-3H-thieno[3,2-d]pyrimidin-4-one

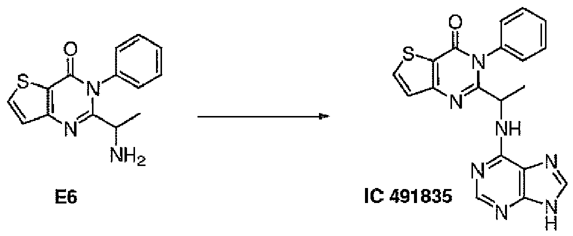

**IC491835: 3- Phenyl-2-[1-(9H-purin-6-ylamino)-ethyl]-3H-thieno[3,2-d]pyrimidin-4-one.** To a solution of 2-(1-Amino-ethyl)-3-phenyl-3H-thieno[3,2-d]pyrimidin-4-one (125 mg, 0.461 mmol) (E6, preparation shown above) in ethanol (10 mL) was added 6-bromopurine (292 mg, 1.47 mmol), and diisopropylethylamine (DIEA) (0.361 mL, 2.07 mmol). The resulting solution was then heated to 85°C in a sealed tube for 4 d, then cooled to room temperature and concentrated. The crude residue was then purified by HPLC (C-18 Luna column 250 x 21.20 mm, 10-20% CH₃CN/H₂Ocontaining 0.05% CF₃CO₂H) product was obtained as a light yellow solid after lyophilizing: ¹H NMR (400 MHz, DMSO-d₆) δ 8. 66 (br s, 1H), 8.36 (br s, 2 H), 8.23 (d, *J* = 5.2 Hz, 1H), 7.60 (m, 3H), 7.46 (m, 3H), 4.88 (m, 1H), 1.46 (d, *J*=7.2 Hz, 3H). LC/MS (AP-ESI, CH₃CO₂H 0.05%) *m*/*z* 390 (MH+).

### Example 6

### Preparation of IC491597: 3-Phenyl-2-(9H-purin-6-ylsulfanylmethyl)-3H-thieno[3,2-dlpyrimidin-4-one

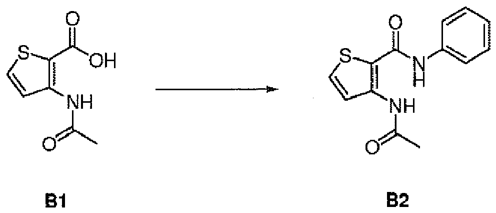

**B2: 3-Acetylamino-thiophene-2-carboxylic acid phenylamide.** General procedure. A mixture of commercially available 3-Acetylamino-thiophene-2-carboxylic acid (3.0 g, 16.2 mmol), *O*-(7-azabenzotriazol-1-yl)-*N,N,N',N*'-tetramethyluronium hexafluorophosphate (HATU) (9.24 g, 24.3 mmol), DIEA (7.05 mL, 40.5 mmol), and aniline (2.21 mL, 24.3 mmol) in dichloromethane (65 mL) was stirred at room temperature for 16 h. The reaction mixture was then concentrated by rotary evaporation to afford a thick viscous residue. The residue was diluted with ethyl acetate (25 mL), then washed with saturated aqueous sodium bicarbonate solution (2 x 50 mL), H₂O (1 x 50 mL), 1 N HCl (2 x 50 mL), and H₂O (1 x 50 mL). The organic extract was then dried over magnesium sulfate, filtered, and concentrated to afford the crude product. Purification by silica gel chromatography (1:1 hexanes/ethyl acetate) afforded purified product. LC/MS (AP-ESI, CH₃CO₂H 0.05%) *m*/*z* 261 (MH+).

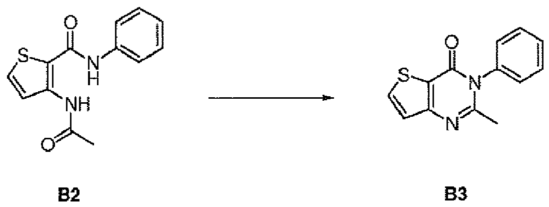

**B3: 2-Methyl-3-phenyl-3*H*-thieno[3,2-*d*]pyrimidin-4-one.** A solution of 3-acetylamino-thiophene-2-carboxylic acid phenylamide (358 mg, 1.38 mmol) in phosphorous oxychloride (7.5 mL) was heated to 105°C in a sealed tube for 1.5 h. The reaction mixture was then cooled to room temperature, and a precipitate formed. The reaction mixture was concentrated by rotary evaporation to afford a residue. The residue was dissolved in ethyl acetate (15 mL), and washed with saturated sodium bicarbonate (2 x 20 mL). The organic extract was then dried over magnesium sulfate, filtered, and concentrated to afford the product without further purification as a brown-orange oil. LC/MS (AP-ESI, CH₃CO₂H 0.05%) *m*/*z* 243 (MH+).

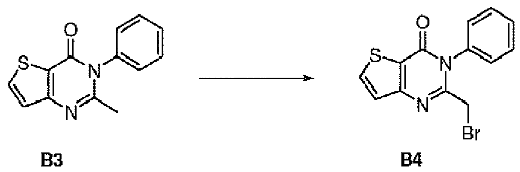

**B4: 2- Bromomethyl-3- phenyl-3*H*-thieno[3,2-d]pyrimidin-4-one.** Following the general procedure described for IC-A7. A stirring solution of 2-methyl-3-phenyl-3*H*-thieno[3,2-*d*]pyrimidin-4-one (100 mg, 0.413 mmol) in acetic acid (3mL) was treated with potassium acetate (61 mg, 0.620 mmol), followed by addition of bromine (32 µL). Purification of the crude product (123 mg) by HPLC (C-18 Vydac column 5.0 x 25 cm, 10-20% CH₃CN/H₂O containing 0.05% CF₃CO₂H), and subsequent lyophilizing afforded purified product as a white solid. LC/MS (AP-ESI, CH₃CO₂H 0.05%) *m*/*z* 323 (MH+).

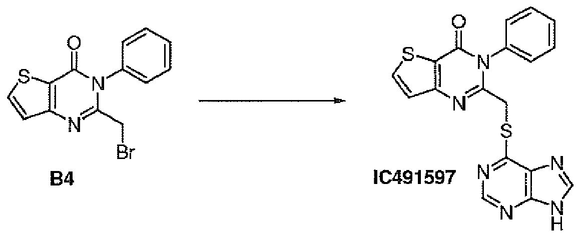

**IC491597: 3-Phenyl-2-(9*H*-purin-6-ylsulfanylmethyl)-3*H-*thieno[3,2-*d*]pyrimidin-4-one.** This compound was prepared by the procedure described for Example 1. A stirring solution of 2-bromomethyl-3-phenyl-3*H-*thieno[3,2-d]pyrimidin-4-one (6 mg, 0,0186 mmol) in DMF (100 µL) was treated with 6-mercaptopurine monohydrate (5 mg, 0.0294 mmol) followed by the addition of potassium carbonate (3 mg, 0.06 mmol). Crude reaction mixture was purified via HPLC (C-18 Luna column 1x18 mm, 10-20% CH₃CN/H₂O containing 0.05% CF₃CO₂H) product was obtained as a fluffy white solid after lyophilizing: ¹H NMR (400 MHz, DMSO-d₆) δ 8.46 (apparent fine d, *J* = 1.8 Hz, 1H), 8.34 (s, 1H), 8.22 (dd, *J* = 2.2 Hz, 5.1 Hz, 1H), 7.49 (m, 6H), 6.56 (s, 1H), 4.40 (apparent fine d, *J* =1.8 Hz); LC/MS (AP-ESI, CH₃CO₂H 0.05%) *m*/*z* 393 (MH+).

### Example 7

### Preparation of 3-Phenyl-2-[1-(9H-purin-6-ylamino)propyl]thieno-[3,2-d]pyrimidin-4(3H)-one

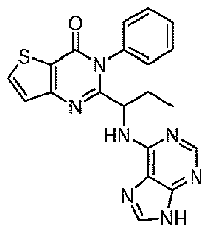

### 7a. Synthesis of tert-Butyl [2-(anilinocarbonyl)-3-thienyl]carbamate.

N,N,N',N'-Tetramethyl-O-(7-azabenzotriazol-1-yl)uronium hexafluorophosphate (7.648 g, 20.12 mmol) was added portion wise to a stirred solution of [A] 3-[(tert-butoxycarbonyl)amino]thiophene-2-carboxylic acid (4.078 g, 16.76 mmol), aniline (2.29 mL, 25.1 mmol) and N,N-diisopropylethylamine (5.84 mL, 33.5 mmol) in dry N,N-dimethylformamide (40.0 mL) under nitrogen. After 90 min ethyl acetate (500ml) was added then washed with water (250ml), sat.NaHCO₃ (200ml), water (300ml), 0.1N.HCl (400ml) and brine (150ml). The organic layer was dried over MgSO₄, filtered and evaporated to give a tan oil. This was chromatographed over 120g SiO₂ eluting with 1500ml 10%ethyl acetate-hexane. The product was obtained as a white solid (4.95g, 92%).

### 7b. Synthesis of 3-Amino-N-phenylthiophene-2-carboxamide.

Trifluoroacetic acid (10.0 mL) was added over ca.1 min to a stirred solution of tert-butyl [2-(anilinocarbonyl)-3-thienyl]carbamate (4.95 g, 15.5 mmol;) in methylene chloride (40.0 mL) then stirred for 1hr. The solvent was then evaporated to give a pale yellow solid which was dissolved in ethyl acetate (250ml) and washed with sat. NaHCO₃ (125ml) and brine (50ml). The organic solution was dried over MgSO₄, filtered and evaporated to give the product as an amber gum (3.4.4g, 100%) which solidified on standing.

### 7c. Synthesis of 3-[(2-Chlorobutanoyl)amino]-N-phenylthiophene-2-carboxamide.

2-Chlorobutanoyl chloride (2.12 mL, 15.8 mmol) was added dropwise over 3-4min to a stirred solution of 3-amino-N-phenylthiophene-2-carboxamide (3,44 g, 15.8 mmol) and triethylamine (3.29 mL, 23.6 mmol) in methylene chloride (40.0 mL) cooled in an ice-acetone bath at -15C. After 60 min. the cooling bath was removed and water (50ml) added followed by CH₂Cl₂ (100ml). The organic layer was washed with 1N.HCl (50ml) and brine (50ml) then dried over MgSO₄. Filtration and evaporation gave a dark amber gum which was chromatographed over 120g of SiO₂. Elution with 10-15%ethyl acetate-hexane gave the product as a pale yellow solid (3.48g, 68%). MS (ESI-) for C₁₅H₁₅ClN₂O₂S *m*/*z* 321.1 (M-H)⁻.

### 7d. Synthesis of 2-(1-Chloropropyl)-3-phenylthieno[3,2-d]pyrimidin-4(3H)-one.

Phosphoryl chloride (50 mL, 500 mmol) was added to 3-[(2-chlorobutanoyl)amino]-N-phenylthiophene-2-carboxamide (3.48 g, 10.8 mmol) in a pressure flask under nitrogen, The tube was sealed and placed in an oilbath and heated to 120°C for 16 hr. The solvent was evaporated and the residue dissolved in EtOAc (250ml) then stirred with sat. NaHCO₃(150ml) for 15min. The organic layer was washed with brine (75ml) and dried over MgSO_{4.} Filtration and evaporation gave a brown oil which was chromatographed over120g of SiO₂. Elution with 5-15%EtOAc-hexane afforded the crude product which was washed with hexane to give a cream solid (2.28g, 69%). MS (ESI+) for C₁₅H₁₃ClN₂OS *m*/*z* 305.1 (M+H)⁺.

### 7e. Synthesis of 2-(1-Aminopropyl)-3-phenylthieno[3,2-d]pyrimidin-4(3H)-one.

2-(1-Chloropropyl)-3-phenylthieno[3,2-d]pyrimidin-4(3H)-one (1.783 g, 5.850 mmol) was placed in a pressure flask under nitrogen and 7 M of ammonia in methanol (40 mL) added. The tube was sealed and heated at 85°C for 48 hr. A solid was filtered and discarded. The filtrate was evaporated and the residue chromatographed over 90g of SiO₂. Elution with 0-15%MeOH-EtOAc afforded the product as a white solid (0.911g, 54%). MS (ESI+) for C₁₅H₁₅N₃OS *m*/*z* 286.2 (M+H)⁺.

### 7f. Synthesis of 3-Phenyl-2-{1-[9-(tetrahydro-pyran-2-yl)-9H-purin-6-ylamino]-propyl}-3H-thieno[3,2-d]pyrimidin-4-one.

N,N-Diisopropylethylamine (0.14 mL, 0.82 mmol) was added to a stirred suspension of 2-(1-aminopropyl)-3-phenylthieno[3,2-d]pyrimidin-4(3H)-one (0.039 g, 0.14 mmol) and 6-chloro-9-(tetrahydro-2H-pyran-2-yl)-9H-purine (0.098 g, 0.41 mmol) in abs. ethanol (3.0 mL) under nitrogen in a pressure tube. The tube was sealed and heated at 85°C for 3 days. The solvent was then evaporated to give an amber gum/glass which was dissolved in EtOAc(20ml). The solution was washed with water (10ml) and brine (10ml) then dried over MgSO₄. Filtration and evaporation gave an amber glass/gum which was chromatographed over 40g of SiO₂. Elution with 0-4%MeOH-EtOAc gave the product as a clear glass (0.039g, 58%). MS (ESI+) for C₂₅H₂₅N₇O₂S *m*/*z* 488.2 (M+H)⁺.

### 7g. Synthesis of 3-Phenyl-2-[1-(9H-purin-6-ylamino)propyl]thieno[3,2-d]pyrimidin-4(3H)-one.

Trifluoroacetic acid (0.30 mL, 3.9 mmol) was added to a stirred solution of 3-phenyl-2-{1-[9-(tetrahydro-pyran-2-yl)-9H-purin-6-ylamino]-propyl}-3H-thieno[3,2-d]pyrimidin-4-one (37 mg, 0.076 mmol) in dry methylene chloride (2.00 mL) then stirred for 1hr, The solvent was then evaporated and the residual amber gum was dissolved in EtOAc (20ml), washed with sat.NaHCO₃ (5ml) and brine (7ml) then dried over MgSO₄. Filtration and evaporation gave a pale amber gum/glass. A little EtOAc was added and a solid formed. Filtration gave the product as an off white solid (19mg, 62%). MS (ESI+) for C₂₀H₁₇N₇OS *m*/*z* 404.1 (M+H)⁺.

### Example 8

### Preparation of 3-(2-Methylphenyl)-2-[1-(9H-purin-6-ylamino)propyl]thieno[3,2-d]-pyrimidin-4(3H)-one

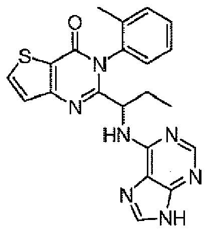

### 8a. Synthesis of tert-Butyl {2-[(2-methylphenyl)carbamoyl]-3-thienyl}carbamate.

Following the procedure in Example 1a starting with 3-[(tert-butoxycarbonyl)amino]thiophene-2-carboxylic acid (2.500 g, 10.28 mmol) and 2-methylaniline (1.64 mL, 15,4 mmol) the product was obtained as a gum (2.658g, 77%) which crystallized on standing.

### 8b. Synthesis of 3-Amino-N-(2-methylphenyl)thiophene-2-carboxamide.

Following the procedure in Example 1b starting with tert-butyl {2-[(2-methylphenyl)carbamoyl]-3-thienyl}carbamate (2.641 g, 7.945 mmol) the product was obtained as a beige solid (1.954g, 100%). MS (ESI-) for C₁₂H₁₂N₂OS *m*/*z* 231.2 (M-H)⁻.

### 8c. Synthesis of 3-[(2-Chlorobutanoyl)amino]-N-(2-methylphenyl)thiophene-2-carboxamide.

Following the procedure in Example 1c and starting with 2-chlorobutanoyl chloride (1.11 mL, 8.26 mmol) and 3-amino-N-(2-methylphenyl)thiophene-2-carboxamide (1.918 g, 8.256 mmol) the product was obtained as a gum which solidified on standing (1.754g, 63%). MS (ESI-) for C₁₆H₁₇ClN₂O₂S *m*/*z* 335.2 (M-H)⁻

### 8d. Synthesis of 2-(1-chloropropyl)-3-(2-methylphenyl)thieno[3,2-d]pyrimidin-4(3H)-one.

Following the procedure for Example 1d and using phosphoryl chloride (20 mL, 200 mmol) and 3-[(2-chlorobutanoyl)amino]-N-(2-methylphenyl)thiophene-2-carboxamide (1.749 g, 5.192 mmol) the product was obtained as a cream solid (1.44g, 87%). MS (ESI+) for C₁₆H₁₅ClN₂OS *m*/*z* 319,1 (M+H)⁺,

### 8e. Synthesis of 2-(1-aminopropyl)-3-(2-methylphenyl)thieno[3,2-d] pyrimidin-4(3H)-one.

Following the procedure from Example 1e and using 2-(1-chloropropyl)-3-(2-methylphenyl)thieno[3,2-d]pyrimidin-4(3H)-one (1.44 g, 4.52 mmol) the product was obtained as a yellow gum (0.346g, 25%), MS (ESI+) for C₁₆H₁₇N₃OS *m*/*z* 300.2 (M+H)⁺.

### 8f. Synthesis of 3-(2-Methylphenyl)-2-(1-{[9-(tetrahydro-2H-pyran-2-yl)-9H-purin-6-yl]amino}propyl)thieno[3,2-d]pyrimidin-4(3H)-one.

Following the procedure from Example 1f and using 2-(1-aminopropyl)-3-(2-methylphenyl)thieno[3,2-d]pyrimidin-4(3H)-one (0.335 g, 1.12 mmol) and 6-chloro-9-(tetrahydro-2H-pyran-2-yl)-9H-purine (0.534 g, 2.24 mmol) the product was obtained as a white foam (0.340g, 60%). MS (ESI+) for C₂₆H₂₇N₇O₂S *m*/*z* 502.2 (M+H)⁺.

### 8g. Synthesis of 3-(2-Methylphenyl)-2-[1-(9H-purin-6-ylamino)propyl]thieno[3,2-d]pyrimidin-4(3H)-one.

Following the procedure of Example 1g and using 3-(2-methylphenyl)-2-(1-{[9-(tetrahydro-2H-pyran-2-yl)-9H-purin-6-yl]amino}propyl)thieno[3,2-d]pyrimidin-4(3H)-one (0.337 g, 0.672 mmol) the product was obtained as an off white solid (0.180g, 64%). MS (ESI+) for C₂₁H₁₉N₇OS *m*/*z* 418.1 (M+H)⁺.

### Example 9

### Preparation of 3-(3-Methylphenyl)-2-[1-(9H-purin-6-ylamino)propyl]thieno[3,2-d]pyrimidin-4(3H)-one

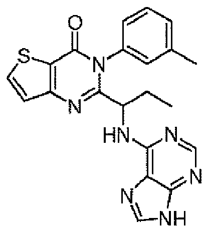

### 9a. Synthesis of tert-Butyl {2-[(3-methylphenyl)carbamoyl]-3-thienyl}carbamate

Following the procedure in Example 1a and using 3-[(tert-butoxycarbonyl)amino]thiophene-2-carboxylic acid (2.500 g, 10.28 mmol), the product was obtained as a white solid (3.21g, 94%).

### 9b. Synthesis of 3-Amino-N-(3-methylphenyl)thiophene-2-carboxamide.

Following the procedure from Example 1b and using tert-butyl {2-[(3-methylphenyl)carbamoyl]-3-thienyl}carbamate (3.21 g, 9.66 mmol) the product was obtained as an amber gum (2.33g, 100%). MS (ESI+) for C₁₂H₁₂N₂OS *m*/*z* 233.2 (M+H)⁺.

### 9c. Synthesis of 3-[(2-Chlorobutanoyl)amino]-N-(3-methylphenyl)thiophene-2-carboxamide

Following the procedure from 1c and using 3-amino-N-(3-methylphenyl)thiophene-2-carboxamide (2.330 g, 10.03 mmol) the product was obtained as a yellow solid (2.248g, 66%).MS (ESI-) for C₁₆H₁₇ClN₂O₂S *m*/*z* 335.2 (M-H)⁻.

### 9d. Synthesis of 2-(1-chloropropyl)-3-(3-methylphenyl)thieno[3,2-d]pyrimidin-4(3H)-one.

Following the procedure in Example 1d and using 3-[(2-chlorobutanoyl)amino]-N-(3-methylphenyl)thiophene-2-carboxamide (2.243 g, 0.006659 mol) the product was obtained as a dark cream solid (1.47g, 69%). MS (ESI+) for C₁₆H₁₅ClN₂OS *m*/*z* 319.1 (M+H)⁺.

### 9e. Synthesis of 2-(1-Aminopropyl)-3-(3-methylphenyl)thieno[3,2-d]pyrimidin-4(3H)-one.

Following the procedure in Example 1e and using 2-(1-chloropropyl)-3-(3-methylphenyl)thieno[3,2-d]pyrimidin-4(3H)-one (1.47 g, 0.00461 mol) the product was obtained as a tan solid (0.48g, 34%). MS (ESI+) for C₁₆H₁₇N₃OS *m*/*z* 300.2 (M+H)⁺.

### 9f. Synthesis of 3-(3-methylphenyl)-2-(1-{[9-(tetrahydro-2H-pyran-2-yl)-9H-purin-6-yl]amino}propyl)thieno[3,2-d]pyrimidin-4(3H)-one.

Following the procedure in Example 1f and using 2-(1-aminopropyl)-3-(3-methylphenyl)thieno[3,2-d]pyrimidin-4(3H)-one (0.466 g, 1.56 mmol) and 6-chloro-9-(tetrahydro-2H-pyran-2-yl)-9H-purine (0.743 g, 3.11 mmol) the product was obtained as a white foam (0.619g, 79%). MS (ESI+) for C₂₆H₂₇N₇O₂S *m*/*z* 502.2 (M+H)⁺.

### 9g. Synthesis of 3-(3-methylphenyl)-2-[1-(9H-purin-6-ylamino)propyl]thieno[3,2-d]pyrimidin-4(3H)-one.

Following the procedure from Example 1g and using 3-(3-methylphenyl)-2-(1-{[9-(tetrahydro-2H-pyran-2-yl)-9H-purin-6-yl]amino}propyl)thieno[3,2-d]pyrimidin-4(3H)-one (0.611 g, 1.22 mmol) the product was obtained as a white solid (0.443g, 87%). MS (ESI+) for C₂₁H₁₉N₇OS *m*/*z* 418.1 (M+H)⁺.

### Example 10

### Preparation of 3-(4-methylphenyl)-2-[1-(9H-purin-6-ylamino)propyl]thieno[3,2-d]pyrimidin-4(3H)-one

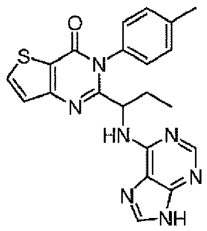

### 10a. Synthesis of tert-Butyl {2-[(4-methylphenyl)carbamoyl]-3-thienyl}carbamate.

Following the procedure from Example 1a and using 3-[(tert-butoxycarbonyl)amino]thiophene-2-carboxylic acid (2.000 g, 8.221 mmol) and p-toluidine (1.32 g, 12.3 mmol) the product was obtained as a white solid (2.61g, 95%).

### 10b. Synthesis of 3-Amino-N-(4-methylphenyl)thiophene-2-carboxamide.

Following the procedure from Example 1b and using tert-butyl {2-[(4-methylphenyl)carbamoyl]-3-thienyl}carbamate (2.598 g, 7.815 mmol) the product was obtained as an amber gum (1.82g, 100%).

### 10c. Synthesis of 3-[(2-Chlorobutanoyl)amino]-N-(4-methylphenyl)thi ophene-2-carboxamide.

Following the procedure from Example 1c and using 2-chlorobutanoyl chloride (1.05 mL, 7.83 mmol) and 3-amino-N-(4-methylphenyl)thiophene-2-carboxamide (1.82 g, 7.83 mmol) the product was obtained as a yellow gum which slowly crystallized on standing (1.914g, 72%). MS (ESI-) for C₁₆H₁₇ClN₂O₂S *m*/*z* 335.1 (M-H)⁻.

### 10d. Synthesis of 2-(1-Chloropropyl)-3-(4-methylphenyl)thieno[3,2-d]pyrimidin-4(3H)-one.

Following the procedure in Example 1d and using 3-[(2-chlorobutanoyl)amino]-N-(4-methylphenyl)thiophene-2-carboxamide (1.906 g, 5.66 mmol) the product was obtained as a yellow foam (1.487g, 82%). MS (ESI+) for C₁₆H₁₅ClN₂OS *m*/*z* 319.1 (M+H)⁺.

### 10e. Synthesis of 2-(1-Aminopropyl)-3-(4-methylphenyl)thieno[3,2-d] pyrimidin-4(3H)-one.

Following the procedure of Example 1e and using 2-(1-chloropropyl)-3-(4-methylphenyl)thieno[3,2-d]pyrimidin-4(3H)-one (1.41 g, 4.42 mmol) the product was obtained as a pale yellow solid (0.344g, 26%). MS (ESI+) for C₁₆H₁₇N₃OS *m*/*z* 300.2 (M+H)⁺.

### 10f. Synthesis of 3-(4-Methylphenyl)-2-(1-{[9-(tetrahydro-2H-pyran- 2-yl)-9H-purin-6-yl]amino}propyl)thieno[3,2-d]pyrimidin-4(3H)-one.

Following the procedure from Example 1f using 2-(1-aminopropyl)-3-(4-methylphenyl)thieno[3,2-d]pyrimidin-4(3H)-one (0.343 g, 1.14 mmol) and 6-chloro-9-(tetrahydro-2H-pyran-2-yl)-9H-purine (0.547 g, 2.29 mmol) the product was obtained as a white foam (0.492g, 85%). MS (ESI+) for C₂₆H₂₇N₇O₂S *m*/*z* 502.2 (M+H)⁺.

### 10g. Synthesis of 3-(4-Methylphenyl)-2-[1-(9H-purin-6-ylamino)propyl]thieno[3,2-d]pyrimidin-4(3H)-one.

Following the procedure of Example 1g and using 3-(4-methylphenyl)-2-(1- {[9-(tetrahydro-2H-pyran-2-yl)-9H-purin-6-yl]amino}propyl)thieno[3,2-d]pyrimidin-4(3H)-one (0.487 g, 0.971 mmol) the product was obtained as a cream solid (0.348g, 85%). MS (ESI+) for C₂₁H₁₉N₇OS *m*/*z* 418.1 (M+H)⁺.

### Example 11

### Preparation of 3-(3,5-Difluorophenyl)-2-[1-(9H-purin-6-ylamino)propyl]thieno[3,2-d] pyrimidin-4(3H)-one

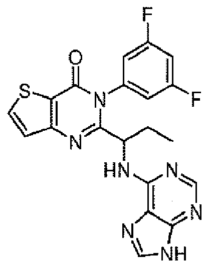

### 11a. Synthesis of tert-Butyl {2-[(3,5-difluorophenyl)carbamoyl]-3-thienyl}carbamate.

Following the procedure from Example 1a and using 3-[(tert-butoxycarbonyl)amino]thiophene-2-carboxylic acid (2.000 g, 8.221 mmol) and [B] 3,5-difluoroaniline (2.65g, 20.5 mmol) for 6 days the product was obtained as a yellow solid (1.447g 49%).

### 11b. Synthesis of 3-Amino-N-(3,5-difluorophenyl)thiophene-2-carboxamide.

Following the procedure from Example 1b and using tert-butyl {2-[(3,5-difluorophenyl)carbamoyl]-3-thienyl} carbamate (1.435 g, 4.049 mmol) the product was obtained as a deep yellow solid (1.012g, 98%). MS (ESI-) for C₁₁H₈F₂N₂OS *m*/*z* 253.2 (M-H)⁻.

### 11c. Synthesis of 3-[(2-Chlorobutanoyl)amino]-N-(3,5-difluorophenyl)thiophene-2-carboxamide.

Following the procedure in Example 1c and using 3-amino-N-(3,5-difluorophenyl)thiophene-2-carboxamide (1.01 g, 3.97 mmol) the product was obtained as a yellow solid 0.976g, 68%). MS (ESI-) for C₁₅H₁₃ClF₂N₂O₂S *m*/*z* 357.1 (M-H)⁻.

### 11d. Synthesis of 2-(1-Chloropropyl)-3-(3,5-difluorophenyl)thieno[3,2-d]pyrimidin-4(3H)-one.

Chlorotrimethylsilane (6.00 mL, 47.3 mmol) was added to a yellow solution of 3-[(2-chlorobutanoyl)amino]-N-(3,5-difluorophenyl)thiophene-2-carboxamide (0.913 g, 2.54 mmol) in acetonitrile (30.0 mL) and triethylamine (18.00 mL, 129.1 mmol) under nitrogen in a pressure flask. The sealed flask was placed in an oilbath and heated to 85°C for 3.5hr. After cooling the solvent was removed on a rotary evaporator and the residue partitioned between water (50ml) and chloroform (50ml). The organic layer was washed with brine (25ml) and dried over MgSO₄. Filtration and evaporation gave a tan solid which was chromatographed over 90g of SiO₂. Elution with 10-20%ethyl acetate-hexane afforded the product as a white solid (0.660g, 76%). MS (ESI+) for C₁₅H₁₁ClF₂N₂OS *m*/*z* 341.0 (M+H)⁺.

### 11e. Synthesis of 2-(1-Aminopropyl)-3-(3,5-difluorophenyl)thieno[3,2-d]pyrimidin-4(3H)-one.

2-(1-Chloropropyl)-3-(3,5-difluorophenyl)thieno[3,2-d]pyrimidin-4(3H)-one (0.650 g, 1.91 mmol) was placed in a pressure flask under nitrogen and 7 M of Ammonia in Methanol (25 mL) added. The flask was sealed and placed in a preheated 85C oil bath and stirred for 3days. The solvent was then evaporated and the residue stirred with chloroform (25ml). The suspension was filtered and the filtrate chromatographed over 90g of SiO₂. Elution with 0-10% MeOH-EtOAc afforded the product as a pale yellow solid (0.307g, 50%). MS (ESI+) for C₁₅H₁₃F₂N₃OS *m*/*z* 322.1 (M+H)⁺.

### 11f. Synthesis of 3-(3,5-Difluorophenyl)-2-(1-{[9-(tetrahydro-2H-pyran-2-yl)-9H-purin-6-yl]amino}propyl)thieno[3,2-d]pyrimidin-4(3H)-one.

N,N-Diisopropylethylamine (0.656 mL, 3.76 mmol) was added to a stirred suspension of 2-(1-aminopropyl)-3-(3,5-difluorophenyl)thieno[3,2-d]pyrimidin-4(3H)-one (0.242 g, 0.753 mmol) and 6-chloro-9-(tetrahydro-2H-pyran-2-yl)-9H-purine (0.359 g, 1.51 mmol) in ethanol (7.0 mL) in a small pressure tube under nitrogen. The tube was sealed and placed in an oilbath at 85C for 22hr. The solvent was then evaporated and the residue dissolved in EtOAc (75ml), washed with water (30ml) and brine (25ml). The solution was dried over MgSO₄, filtered and evaporated to give an amber gum. Chromatography over 90g of SiO₂ eluting with 0-4% MeOH-EtOAc gave the product as a pale yellow foam (0.385g, 97%). MS (ESI+) for C₂₅H₂₃F₂N₇O₂S *m*/*z* 524.2 (M+H)⁺.

### 11g. Synthesis of 3-(3,5-Difluorophenyl)-2-[1-(9H-purin-6-ylamino)propyl]thieno[3,2-d] pyrimidin-4(3H)-one.

Trifluoroacetic acid (2.0 mL) was added over 5-10sec to a stirred pale yellow solution of 3-(3,5-difluorophenyl)-2-(1-{[9-(tetrahydro-2H-pyran-2-yl)-9H-purin-6-yl]amino}propyl)thieno[3,2-d]pyrimidin-4(3H)-one (0.376 g, 0.718 mmol) in methylene chloride (10.0 mL) then stirred for 75min. The solvent was then evaporated and the dark residue partitioned between EtOAc (100mL) and sat.NaHCO₃ (50mL), washed with brine (30mL) and dried over MgSO₄. Filtration and evaporation gave the product as an off white solid (0.250g, 79%). MS (ESI+) for C₂₀H₁₅F₂N₇OS *m*/*z* 440.1 (M+H)⁺. ¹H NMR (CDCl₃/CD₃OD) δ 0.93 (t, 3 H), 1.95 (m, 2 H), 5.00 (m, 1 H), 6.95 (m, 1 H), 7.00 (m, 1 H), 7.20 (m, 1 H), 7.30 (d, 1 H), 7.85 (d, 1 H), 7.91 (s, 1 H), 8.19 (s, 1 H).

### Example 12

### Preparation of 3-(3-Methoxyphenyl)-2-[1-(9H-purin-6-ylamino)propyl]thieno[3,2-d]pyrimidin-4(3H)-one

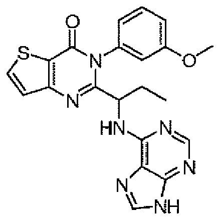

### 12a. Synthesis of tert-Butyl {2-[(3-methoxyphenyl)carbamoyl]-3-thienyl}carbamate.

Following the procedure from Example 1a and using 3-[(tert-butoxycarbonyl)amino]thiophene-2-carboxylic acid (2.000 g, 8.221 mmol) and 3-methoxyaniline (1.38 mL, 12.3 mmol) the product was obtained as a white solid (2.636g, 92%).

### 12b. Synthesis of 3-Amino-N-(3-methoxyphenyl)thiophene-2-carboxamide.

Following the procedure from Example 1b and using trifluoroacetic Acid (5.0 mL, 65 mmol) and tert-butyl {2-[(3-methoxyphenyl)carbamoyl]-3-thienyl}carbamate (2.63 g, 7.55 mmol) the product was obtained as an amber gum (1.99g, 100%) which slowly solidified.

### 12c. Synthesis of 3-[(2-Chlorobutanoyl)amino]-N-(3-methoxyphenyl)thiophene-2-carboxamide.

Following the procedure from Example 1c and using 2-chlorobutanoyl chloride (1.06 mL, 7.93 mmol) and 3-amino-N-(3-methoxyphenyl)thiophene-2-carboxamide (1.97 g, 7.93 mmol) the product was obtained as a pale yellow gum (1.743g, 62%) which crystallized on standing. MS (ESI-) for C₁₆H₁₇ClN₉O₃S *m*/*z* 315.2 (M-H)⁻.

### 12d. Synthesis of 2-(1-Chloropropyl)-3-(3-methoxyphenyl)thieno[3,2- d]pyrimidin-4(3H)-one.

Following the procedure from Example 5d and using 3-[(2-chlorobutanoyl)amino]-N-(3-methoxyphenyl)thiophene-2-carboxamide (1.635 g, 4.634 mmol) the product was obtained as a cream solid (0.919g, 59%). MS (ESI+) for C₁₆H₁₅ClN₂O₂S *m*/*z* 335.1 (M+H)⁺.

### 12e. Synthesis of 2-(1-Aminopropyl)-3-(3-methoxyphenyl)thieno[3,2-d ]pyrimidin-4(3H)-one.

Following the procedure from Example 1e and using 2-(1-chloropropyl)-3-(3-methoxyphenyl)thieno[3,2-d]pyrimidin-4(3H)-one (0.915 g, 2.73 mmol) the product was obtained as a lime-yellow foam (0.309g, 35%). MS (ESI+) for C₁₆H₁₇N₃O₂S *m*/*z* 316.2 (M+H)⁺.

### 12f. Synthesis of 3-(3-methoxyphenyl)-2-(1-{[9-(tetrahydro-2H-pyran-2-yl)-9H-purin-6-yl]amino}propyl)thieno[3,2-d]pyrimidin-4(3H)-one.

Following the procedure in Example 1f and using 2-(1-aminopropyl)-3-(3-methoxyphenyl)thieno[3,2-d]pyrimidin-4(3H)-one (0.306 g, 0.970 mmol) and 6-chloro-9-(tetrahydro-2H-pyran-2-yl)-9H-purine (0.463 g, 1.94 mmol) the product was obtained as a white foam (0.450g, 89%). MS (ESI+) for C₂₆H₂₇N₇O₃S *m*/*z* 518.2 (M+H)⁺.

### 12g. Synthesis of 3-(3-Methoxyphenyl)-2-[1-(9H-purin-6-ylamino)propyl]thieno[3,2-d]pyrimidin-4(3H)-one.

Following the procedure in Example 1g and using 3-(3-methoxyphenyl)-2-(1-{[9-(tetrahydro-2H-pyran-2-yl)-9H-purin-6-yl]amino}propyl)thieno[3,2-d]pyrimidin-4(3H)-one (0.449 g, 0.867 mmol) the product was obtained as a white solid (0.229g, 61%). MS (ESI+) for C₂₁H₁₉N₇O₂S *m*/*z* 434.1 (M+H)⁺. ¹H NMR (CDCl₃) δ 0.91 (m, 3 H), 1.86 (m, 1 H), 2.07 (m, 1 H), 3.80 (s, 1.8 H), 3.89 (s, 1.2 H), 5.29 (m, 1 H), 6.64 (m, 1 H), 6.90 (m, 0.4 H), 6.97 (m, 0.6 H), 7.09 (m, 2 H), 7.35 (m, 1 H), 7.52 (m, 1 H), 7.81 (m, 1 H), 7.99 (s, 1 H), 8.33 (s, 1 H), 12.93 (s, 1 H).

### Example 13

### Preparation of 2-{1-[(2-Amino-9H-purin-6-yl)amino]propyl}-3-phenylthieno[3,2-d]pyrimidin-4(3H)-one

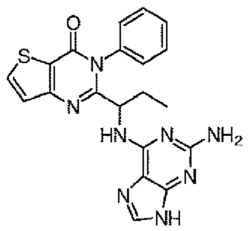

A stirred suspension of 2-(1-aminopropyl)-3-phenylthieno[3,2-d]pyrimidin-4(3H)-one (0.100 g, 0.350 mmol) and 6-bromo-9H-purin-2-amine (0.112 g, 0.526 mmol) in isopropyl alcohol (4.00 mL) containing N,N-diisopropylethylamine (0.366 mL, 2.10 mmol) was placed in a microwave reactor at 160C for 2hr. Upon cooling, the suspension was filtered and evaporated. The residue was partitioned between EtOAc (50ml) and water (15ml). The organic layer was washed with brine (15ml) and dried over MgSO₄, Filtration and evaporation gave a white solid which was chromatographed over 40g of SiO₂ eluting with 0-6% MeOH (containing 10% aqueous conc.NH₄OH)-CHCl₃. The product was obtained as an off white solid (0.098g, 67%). MS (ESI+) for C₂₀H₁₈N₈OS *m*/*z* 419.1 (M+H)⁺. ¹H NMR (CDCl₃/CD₃OD) δ 0.79 (t, 3 H), 1.74 (m, 1 H), 1.85 (m, 1 H), 4.98 (m, 1 H), 7.29 (m, 2 H), 7.39 (m, 1 H), 7.54 (m, 4 H), 7.79 (d, 1 H).

### Example 14

### Preparation of 2-{1-[(2-Fluoro-9H-purin-6-yl)amino]propyl}-3-phenylthieno[3,2-d] pyrimidin-4(3H)-one

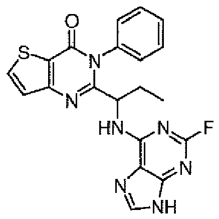

### 14a. Synthesis of 2-{1-{[2-Fluoro-9-(tetrahydro-2H-pyran-2-yl)-9H-purin-6-yl]amino} propyl)-3-phenylthieno[3,2-d]pyrimidin-4(3H)-one.

N,N-Diisopropylethylamine (0.763 mL, 4.38 mmol) was added to a stirred suspension of 2-(1-aminopropyl)-3-phenylthieno[3,2-d]pyrimidin-4(3H)-one (0.250 g, 0.876 mmol) and 6-chloro-2-fluoro-9-(tetrahydro-2H-pyran-2-yl)-9H-purine (0.337 g, 1.31 mmol) in abs. ethanol (7.00 mL) in a small pressure flask under nitrogen. The flask was sealed and heated at 85C for 15hr. The solvent was evaporated and the residue dissolved in EtOAc (75ml) then washed with water (35ml) and brine (25ml). The solution was dried over MgSO₄, filtered and evaporated to give a clear oil which was chromatographed over 90g of SiO₂. Elution with 0-5% MeOH-EtOAc afforded the product as a white foam (0.429g, 96%). MS (ESI+) for C₂₅H₂₄FN₇O₂S *m*/*z* 506.1 (M+H)⁺.

### 14b. Synthesis of 2-{1-[(2-Fluoro-9H-purin-6-yl)amino]propyl}-3-phenylthieno[3,2-d]pyrimidin-4(3H)-one.

Following the procedure from Example 1a but using] 2-(1-{[2-fluoro-9-(tetrabydro-2H-pyran-2-yl)-9H-purin-6-yl]amino}propyl)-3-phenylthieno[3,2-d]pyrimidin-4(3H)-one (0.423 g, 0.837 mmol) the product was obtained as an off white solid (0.270g, 76%). MS (ESI+) for C₂₀H₁₆FN₇OS m/z 422.1 (M+H)⁺. ¹H NMR (CDCl₃/CD₃OD) δ 0.82 (t, 3 H), 1.83 (m, 2 H), 4.78 (m, 1 H), 7.23 (d, 1 H), 7.26 (m, 1 H), 7.52 (m, 3 H), 7.61 (m, 1 H), 7.77 (d, 1 H), 7.85 (s, 1 H).

### Example 15

### Preparation of 2-[(6-Amino-9H-purin-9-yl)methyl]-3-(2-methylphenyl)thieno[3,2-d]pyrimidin-4(3H)-one

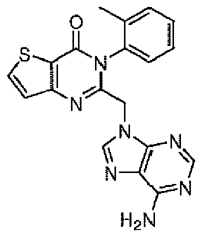

### 15a. Synthesis of 3-[(Chloroacetyl)amino]-N-(2-methylphenyl)thiophene-2-carboxamide.

Chloroacetyl chloride (0.642 mL, 8.08 mmol) was added dropwise over 2-3 min to a stirred pale amber solution of 3-amino-N-(2-methylphenyl)thiophene-2-carboxamide (1.876 g, 8.076 mmol) and triethylamine (1.24 mL, 8.88 mmol) in methylene chloride (25.0 mL) cooled in an ice-acetone bath at -15C. After 1hr. water (25ml) was added followed by methylene chloride (100ml). The organic layer was washed with 1N.HCl (50ml) and brine (25ml) then dried over MgSO₄. Filtration and evaporation gave a dirty yellow solid which was washed with a little ether to afford a tan solid (2.000g, 80%).

### 15b. Synthesis of 2-(Chloromethyl)-3-(2-methylphenyl)thieno[3,2-d]pyrimidin-4(3H)-one.

Following the procedure from Example 5d but using 3-[(chloroacetyl)amino]-N-(2-methylphenyl)thiophene-2-carboxamide (1.00 g, 3.24 mmol), the product was obtained as an amber gum (0.240g, 25%). MS (ESI+) for C₁₄H₁₁ClN₂OS *m*/*z* 291.1 (M+H)⁺.

### 15c. Synthesis of 2-[(6-Amino-9H-purin-9-yl)methyl]-3-(2-methylphenyl)thieno[3,2-d]pyrimidin-4(3H)-one.

Dry N,N-dimethylformamide (2.0 mL) was added to a stirred mixture of adenine (0.0820 g, 0.607 mmol) and sodium hydride (60:40, sodium hydride: mineral oil, 0.0362 g) under nitrogen and the mixture heated at 75C for 20min. A solution of 2-(chloromethyl)-3-(2-methylphenyl)thieno[3,2-d]pyrimidin-4(3H)-one (0.234 g, 0.805 mmol) in dry N,N-dimethylformamide (1.0 mL) was then added and rinsed in with ca.0.5ml of additional dry DMF. The reaction became dark magenta in color. After 1hr the reaction was allowed to cool and EtOAc (100ml) was added. The solution was washed with water (4x50ml) and brine (50ml) and dried over MgSO₄. Filtration and evaporation gave a dirty tan solid. Chromatography over 40g of SiO₂ eluting with 0-10% MeOH-EtOAc afforded the product as a beige solid (0.090g, 38%). MS (ESI+) for C₁₉H₁₅N₇OS *m*/*z* 390.1 (M+H)⁺. ¹H NMR (CDCl₃) δ 2.22 (s, 3 H), 4.95 (d, 1 H), 5.07 (d, 1 H), 5.63 (s, 2 H), 7.25 (m, 2 H), 7.42 (m, 1 H), 7.48 (m, 2 H), 7.81 (d, 1 H), 7.83 (s, 1 H), 8.28 (s, 1 H).

### Example 16

### Preparation of 2-[1-(4-Amino-3-phenyl-1H-pyrazolo[3,4-d]pyrimidin-1-yl)propyl]-3-phenylthieno[3,2-d]pyrimidin-4(3H)-one

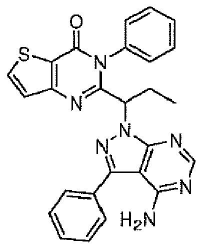

N,N-Dimethylformamide (1.00 mL) was added to a solid stirred mixture of 3-phenyl-1H-pyrazolo[3,4-d]pyrimidin-4-amine (0.0462 g, 0.219 mmol) and sodium hydride in mineral oil (60:40, Sodium hydride: Mineral Oil, 0.0155 g) under nitrogen. The stirred mixture was heated to 75C in an oilbath for 30min then a solution of 2-(1-chloropropyl)-3-phenylthieno[3,2-d]pyrimidin-4(3H)-one (0.100 g, 0.328 mmol) in N,N-Dimethylformamide (0.50 mL) was added rinsed in with an additional 0.2ml of DMF. The heated reaction was stirred for 4hr. then allowed to cool. EtOAc (25ml) was added then washed with water (4x15ml) and brine (15ml). The solution was dried (MgSO₄), filtered, evaporated and the residue chromatographed over 40g of SiO₂. Elution with 0-2% MeOH-EtOAc afforded the product as an off white solid (0.053g, 50%). MS (ESI+) for C₂₆H₂₁N₇OS m/z 480.1 (M+H)⁺. ¹H NMR (CDCl₃) δ 0.91 (t, 3 H), 2.53 (m, 2 H), 5.55 (s, 2 H), 5.80 (m, 1 H), 6.48 (d, 1 H), 6.91 (t, 1 H), 7.23 (t, 1 H), 7.35 (d, 1 H), 7.46 (d, 1 H), 7.54 (m, 4 H), 7.65 (m, 2 H), 7.84 (d, 1 H), 8.06 (s, 1 H).

### Example 17

### Preparation of 2-[(6-Amino-9H-purin-9-yl)methyl]-3-(2-methylphenyl)thieno[2,3-d]pyrimidin-4(3H)-one

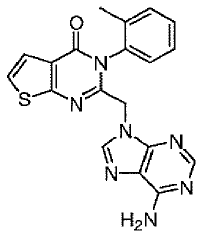

### 17a. Synthesis of Methyl 2-[(tert-butoxycarbonyl)amino]thiophene-3-carboxylate.

Following the procedure from Example 1a but using methyl 2-aminothiophene-3-carboxylate (5.00 g, 31.8 mmol), the product was obtained as a colorless viscous oil 5.768g, 70%).

### 17b. Synthesis of 2-[(tert-Butoxycarbonyl)amino]thiophene-3-carboxylic acid.

A 1.000M solution of lithium hydroxide in water (23.5 mL) was added to a colorless stirred solution of methyl 2-[(tert-butoxycarbonyl)amino]thiophene-3-carboxylate (5.866 g, 22.80 mmol) in tetrahydrofuran (130.0 mL). The reaction was placed under nitrogen in an oilbath and heated to 75C for 48hr then allowed to cool. Most of the THF was evaporated at 35C. Water (100ml) was added then washed with ether (2x100ml). The water layer was cooled in ice, stirred vigorously and conc.H₂SO₄ was added to pH 2. The precipitate was filtered and washed with water (65ml). After drying, a white solid (4.688g, 84%) was obtained.

### 17c. Synthesis of tert-Butyl 13-[(2-methylphenyl)carbamoyl]-2-thienyl}carbamate.

Following the procedure from Example 1c but using 2-[(tert-butoxycarbonyl)amino]thiophene-3-carboxylic acid (2.001 g, 8.225 mmol), the product was obtained as a pale pink solid 2.483g, 90%). MS (ESI+) for C₁₇H₂₀N₂O₃S *m*/*z* 331.1 (M+H)⁺.

### 17d. Synthesis of 2-Amino-N-(2-methylphenyl)thiophene-3-carboxamide hydrochloride.

A 4.00 M solution of hydrogen chloride in 1,4-dioxane (10.0 mL) was added to tert-butyl {3-[(2-methylphenyl)carbamoyl]-2-thienyl carbamate (1.040 g, 3.128 mmol) and the resulting colorless solution stirred at room temp. After 5min a white solid began to precipitate and the reaction slowly developed a pale teal color. After 1.5hr ether (50ml) was added and the reaction filtered to afford the product as a pale green/blue solid (0.902g, 100%).

### 17e. Synthesis of 2-[(Chloroacetyl)amino]-N-(2-methylphenyl)thiophene-3-carboxamide.

2-Amino-N-(2-methylphenyl)thiophene-3-carboxamide hydrochloride (0.900 g, 3.35 mmol) was stirred under nitrogen in dry methylene chloride (12.0 mL) to give a pale blue-green suspension. The stirred mixture was cooled to -15C in an ice-acetone bath and triethylamine (1.634 mL, 11.72 mmol) added. Immediately all went into solution and the color changed to pale pinkish brown. Chloroacetyl chloride (0.2663 mL, 3.349 mmol) was added and a pale grey/green color developed immediately with precipitation. The reaction was removed from cooling bath after 30min when the color was dirty green. Water (10ml) was added after 30min plus CH₂Cl₂ (50ml), Additional water (20ml) and CH₂Cl₂ (50ml) were added and the organic layer was washed with 1N.HCl (50ml) and brine (25ml). After drying over MgSO₄ the solution was filtered and evaporated. The residue was chromatographed over 90g of SiO₂ eluting with 10-25%EtOAc-hexane. The product was obtained as an off white solid 0.498g, 48%). MS (ESI-) for C₁₄H₁₃ClN₂O₂S *m*/*z* 307.2 (M-H)⁻.

### 17f. Synthesis of 2-(Chloromethyl)-3-(2-methylphenyl)thieno[2,3-d]pyrimidin-4(3H)-one.

Following the procedure in Example 5d but using 2-[(chloroacetyl)amino]-N-(2-methylphenyl)thiophene-3-carboxamide (0.250 g, 0.810 mmol) for 30min at 75C, the product was obtained as a clear gum (0.202g, 85%). MS (ESI+) for C₁₄H₁₁ClN₂OS *m*/*z* 291.1 (M+H)⁺.

### 17g. Synthesis of 2-[(6-Amino-9H-purin-9-yl)methyl]-3-(2-methylphenyl)thieno[2,3-d]pyrimidin-4(3H)-one.

Dry N,N-dimethylformamide (2.50 mL) was added to a stirred mixture of adenine (0.139 g, 1.03 mmol) and sodium hydride (60:40, sodium hydride: mineral oil, 0.055 g) under nitrogen and the flask heated to 75C in an oilbath reaching 75C by 8.13am. After 30min a solution of 2-(chloromethyl)-3-(2-methylphenyl)thieno[2,3-d]pyrimidin-4(3H)-one (0.200 g, 0.688 mmol) in dry N,N-dimethylformamide (1.0 mL) was added and rinsed in with ca.0.5ml of additional dry DMF. The reaction became dark magenta in color. After 30min the reaction was allowed to cool EtOAc (100ml) was added. The solution was washed with water (4x50ml) and brine (50ml) then dried over MgSO₄. Filtration and evaporation gave a dirty tan gum which was chromatographed over 40g of SiO₂. Elution with 0-10% MeOH-EtOAc afforded the product as a beige solid (0.077g, 29%). MS (ESI+) for C₁₉H₁₅N₇OS *m*/*z* 390.1 (M+H)⁺. ¹H NMR (CDCl₃) δ 2.22 (s, 3 H), 4.93 (d, 1 H), 5.06 (d, 1 H), 5.61 (s, 2 H), 7.24 (d, 1 H), 7.29 (m, 1 H), 7.42 (m, 1 H), 7.50 (m, 3 H), 7.82 (s, 1 H), 8.29 (s, 1 H).

The following enumerated embodiments further illustrate various aspects of the present invention, without limiting its scope:
1. A compound of the formula (1): 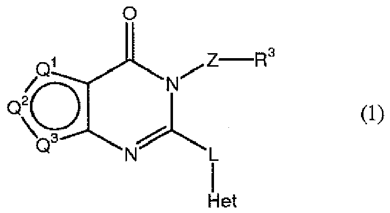 wherein:
   one of Q¹, Q² and Q³ is S, and the other of two of Q¹, Q² and Q³ are -CR¹-;
      wherein each R¹ is independently H, halo, OR, NR₂, NROR, NRNR₂, SR, SOR, SO₂R, SO₂NR₂, NRSO₂R, NRCONR₂, NRCOOR, NRCOR, CF₃, CN, COOR, CONR₂, OOCR, COR, or NO₂,
      or R¹ can be an optionally substituted member selected from the group consisting of C1-C8 alkyl, C2-C8 heteroalkyl, C2-C8 alkenyl, C2-C8 heteroalkenyl, C2-C8 alkynyl, C2-C8 heteroalkynyl, C1-C8 acyl, C2-C8 heteroacyl, C6-C10 aryl, C5-C12 heteroaryl, C7-C12 arylalkyl, and C6-C12 heteroarylalkyl groups,
      wherein each R is independently H or C1-C8 alkyl, C2-C8 heteroalkyl, C2-C8 alkenyl, C2-C8 heteroalkenyl, C2-C8 alkynyl, C2-C8 heteroalkynyl, C1-C8 acyl, C2-C8 heteroacyl, C6-C10 aryl, C5-C10 heteroaryl, C7-C12 arylalkyl, or C6-C12 heteroarylalkyl,
      and wherein two R on the same atom or on adjacent atoms can be linked to form a 3-8 membered ring, optionally containing one or two N, O or S as ring members;
      and wherein each R group other than H, and each ring formed by linking two R groups together, is optionally substituted;
   Z is a bond, or is O, NR², C1-C6 alkylene or C1-C6 heteroalkylene, each of which is optionally substituted with up to two C1-C6 alkyl or C2-C6 heteroalkyl groups, where two of said alkyl or heteroalkyl groups can optionally cyclize to form a 3-7 membered ring containing up to two heteroatoms selected from O, N and S as ring members;
   R³ is aryl, heteroaryl, cycloalkyl, or heterocycloalkyl, each of which is optionally substituted with up to three R¹,
      or R³ can be H if Z is not a bond;
   L is selected from the group consisting of -C(R²)₂-, -C(R²)₂-C(R²)₂-, -C(R²)₂-NR²- , and -C(R²)₂-S(O)ₙ-,
      wherein each R² is independently H or an optionally substituted member selected from C1-C6 alkyl, C2-C6 heteroalkyl, C2-C6 alkenyl, and C2-C6 alkynyl, and n is 0-2;
      and two R², if present on L, can cyclize to form a 3-7 membered ring that may contain up to two heteroatoms selected from N, O and S as ring members;
   Het is a monocyclic or bicyclic ring system wherein at least two ring atoms are N and wherein at least one ring is aromatic, and Het is optionally substituted with up to three substituents selected from R⁴, N(R⁴)₂, S(O)ₚR⁴, OR⁴, halo, CF₃, CN, NR⁴OR⁴, NR⁴N(R⁴)₂, SR⁴, SOR⁴, SO₂R⁴, SO₂N(R⁴)₂, NR⁴SO₂R⁴, NR⁴CON(R⁴)₂, NR⁴COOR⁴, NR⁴COR⁴, CN, COOR⁴, CON(R⁴)₂, OOCR⁴, COR⁴, or NO₂,
      wherein each R⁴ is independently H or an optionally substituted member selected from the group consisting of C1-C8 alkyl, C2-C8 heteroalkyl, C2-C8 alkenyl, C2-C8 heteroalkenyl, C2-C8 alkynyl, C2-C8 heteroalkynyl, C1-C8 acyl, C2-C8 heteroacyl, C6-C10 aryl, C5-C10 heteroaryl, C7-C12 arylalkyl, and C6-C12 heteroarylalkyl,
      and wherein two R⁴ on the same atom or on adjacent atoms can be linked to form a 3-8 membered ring, optionally containing one or two heteroatoms selected from N, O and S;
      wherein the optional substituents on each optionally substituted alkyl, heteroalkyl, alkenyl, heteroalkenyl, alkynyl, heteroalkynyl, acyl, heteroacyl, aryl, heteroaryl, arylalkyl and heteroarylalkyl are selected from C1-C4 alkyl, halo, CF₃, CN, =O, =N-CN, =N-OR', =NR', OR', NR'₂, SR', SO₂R', SO₂NR'₂, NR'SO₂R', NR'CONR'₂, NR'COOR', NR'COR', CN, COOR', CONR'₂, OOCR', COR', and NO₂,
      wherein each R' is independently H, C1-C6 alkyl, C2-C6 heteroalkyl, C1-C6 acyl, C2-C6 heteroacyl, C6-C10 aryl, C5-C10 heteroaryl, C7-12 arylalkyl, or C6-12 heteroarylalkyl, each of which is optionally substituted with one or more groups selected from halo, C1-C4 alkyl, C1-C4 heteroalkyl, C1-C6 acyl, C1-C6 heteroacyl, hydroxy, amino, and =O;
      and wherein two R' on the same or adjacent atoms can be linked to form a 3-7 membered ring optionally containing up to three heteroatoms selected from N, O and S; and
      p is 0-2;
   or a pharmaceutically acceptable salt thereof.
2. The compound of embodiment 1, wherein Q¹ is S.
3. The compound of embodiment 1, wherein Q² is S.
4. The compound of embodiment 1, wherein Q³ is S.
5. The compound of any of embodiments 1-4, wherein Z is a bond and R³ is optionally substituted aryl.
6. The compound of embodiment 5, wherein Het is an optionally substituted bicyclic group consisting of two aromatic rings fused together, wherein each of the two aromatic rings contains at least one N as a ring member.
7. The compound of embodiment 6, wherein Het represents a purine ring system.
8. The compound of embodiment 6, wherein Het represents a pyrazolopyrimidine ring system.
9. The compound of embodiment 6, wherein Het represents a pyrrolopyrimidine ring system.
10. The compound of any of embodiments 6-9, wherein L is CHR².
11. The compound of any of embodiments 6-9, wherein L is -CHR²-NR²-.
12. The compound of any of embodiments 6-9, wherein L is -CHR²-S(O)ₙ-, and n is 0 or 2.
13. The compound of any of embodiments 10-12, wherein L contains a chiral center that is in the S stereochemical configuration.
14. The compound of any of embodiments 1-11, or a pharmaceutically acceptable salt thereof.
15. A method to treat a hematologic cancer, comprising administering to a subject diagnosed with a hematologic cancer an effective amount of a compound of any of embodiments 1-14.
16. The method of embodiment 15, wherein the hematologic cancer is leukemia.
17. The method of embodiment 16, wherein the leukemia is acute lymphoblastic leukemia; acute myeloid leukemia; chronic lymphocytic leukemia; chronic myelogenous leukemia; or hairy cell leukemia.
18. A method to treat an inflammatory disorder or immune disorder, comprising administering to a subject diagnosed with an inflammatory disorder or immune disorder an effective amount of a compound of any of embodiments 1-14.
19. The method of embodiment 18, wherein the inflammatory disorder or immune disorder is rheumatoid arthritis, multiple sclerosis, asthma, systemic lupus erythematosus, scleroderma, Sjögren's syndrome, myasthenia gravis, Guillain-Barré syndrome, Hashimoto's thyroiditis, Graves' disease, inflammatory bowel disease (IBD), Crohn's disease, ulcerative colitis, vasculitis, hemolytic anemia, thrombocytopenia, psoriasis, type I (insulin dependent) diabetes, or allergic rhinitis.
20. A method to treat hypertension, comprising administering to a subject diagnosed with hypertension an effective amount of a compound of any of embodiments 1-14.
21. A pharmaceutical composition comprising a compound of any of embodiments 1-14, admixed with at least one pharmaceutically acceptable excipient.
22. The pharmaceutical composition of embodiment 21, which is a solid dosage form for oral administration.
23. Use of a compound of any of embodiments 1-14 for the manufacture of a medicament.
24. The use of embodiment 23, wherein the medicament is a medicament for the treatment of a hematological cancer or an immune disorder or hypertension.

The foregoing examples are illustrative only, and are not intended to limit the scope of the invention. Each publication mentioned herein is expressly incorporated by reference. References made herein to various publications do not indicate that such references are prior art.

## Claims

1. A compound of formula (1): 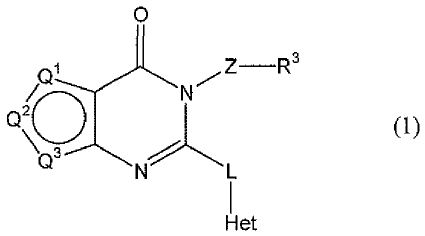 wherein:
one of Q¹, Q² and Q³ is S, and the other of two of Q¹, Q² and Q³ are -CR¹-;
wherein each R¹ is independently H, halo, OR, NR₂, NROR, NRNR₂, SR, SOR, SO₂R, SO₂NR₂, NRSO₂R, NRCONR₂, NRCOOR, NRCOR, CF₃, CN, COOR, CONR₂, OOCR, COR, or NO₂,
or R¹ can be an optionally substituted member selected from the group consisting of C1-C8 alkyl, C2-C8 heteroalkyl, C2-C8 alkenyl, C2-C8 heteroalkenyl, C2-C8 alkynyl, C2-C8 heteroalkynyl, C1-C8 acyl, C2-C8 heteroacyl, C6-C10 aryl, C5-C12 heteroaryl, C7-C12 arylalkyl, and C6-C12 heteroarylalkyl groups,
wherein each R is independently H or C1-C8 alkyl, C2-C8 heteroalkyl, C2-C8 alkenyl, C2-C8 heteroalkenyl, C2-C8 alkynyl, C2-C8 heteroalkynyl, C1-C8 acyl, C2-C8 heteroacyl, C6-C10 aryl, C5-C10 heteroaryl, C7-C12 arylalkyl, or C6-C12 heteroarylalkyl,
and wherein two R on the same atom or on adjacent atoms can be linked to form a 3-8 membered ring, optionally containing one or two N, O or S as ring members;
and wherein each R group other than H, and each ring formed by linking two R groups together, is optionally substituted;
Z is a bond, or is O, NR², C1-C6 alkylene or C1-C6 heteroalkylene, each of which is optionally substituted with up to two C1-C6 alkyl or C2-C6 heteroalkyl groups, where two of said alkyl, or heteroalkyl groups can optionally cyclize to form a 3-7 membered ring containing up to two heteroatoms selected from O, N and S as ring members;
R³ is aryl, heteroaryl, cycloalkyl, or heterocycloalkyl, each of which is optionally substituted with up to three R¹,
or R³ can be H if Z is not a bond;
L is selected from the group consisting of -C(R²)₂-, -C(R²)₂-C(R²)₂-, -C(R²)₂-NR²-, and -C(R²)₂-S(O)ₙ-,
wherein each R² is independently H or an optionally substituted member selected from C1-C6 alkyl, C2-C6 heteroalkyl, C2-C6 alkenyl, and C2-C6 alkynyl, and n is 0-2; and two R², if present on L, can cyclize to form a 3-7 membered ring that may contain up to two heteroatoms selected from N, O and S as ring members;
Het is a monocyclic aromatic ring system, wherein at least two ring atoms are N, and Het is optionally substituted with up to three substituents selected from R⁴, N(R⁴)₂, S(O)ₚR⁴, OR⁴, halo, CF₃, CN, NR⁴OR⁴, NR⁴N(R⁴)₂, SR⁴, SOR⁴, SO₂R⁴, SO₂N(R⁴)₂, NR⁴SO₂R⁴, NR⁴CON(R⁴)₂, NR⁴COOR⁴, NR⁴COR⁴, CN, COOR⁴, CON(R⁴)₂, OOCR⁴, COR⁴, or NO₂,
wherein each R⁴ is independently H or an optionally substituted member selected from the group consisting of C1-C8 alkyl, C2-C8 heteroalkyl, C2-C8 alkenyl, C2-C8 heteroalkenyl, C2-C8 alkynyl, C2-C8 heteroalkynyl, C1-C8 acyl, C2-C8 heteroacyl, C6-C10 aryl, C5-C10 heteroaryl,
C7-C12 arylalkyl, and C6-C12 heteroarylalkyl,
and wherein two R⁴ on the same atom or on adjacent atoms can be linked to form a 3-8 membered ring, optionally containing one or two heteroatoms selected from N, O and S;
wherein the optional substituents on each optionally substituted alkyl, heteroalkyl, alkenyl, heteroalkenyl, alkynyl, heteroalkynyl, acyl, heteroacyl, aryl, heteroaryl, arylalkyl and heteroarylalkyl are selected from C1-C4 alkyl, halo, CF₃, CN, =O, =N-CN, =N-OR', =NR', OR', NR'₂, SR', SO₂R', SO₂NR'₂, NR'SO₂R', NR'CONR'₂, NR'COOR', NR'COR', CN, COOR', CONR'₂, OOCR', COR', and NO₂,
wherein each R' is independently H, C1-C6 alkyl, C2-C6 heteroalkyl, C1-C6 acyl, C2-C6 heteroacyl, C6-C10 aryl, C5-C10 heteroaryl, C7-12 arylalkyl, or C6-12 heteroarylalkyl, each of which is optionally substituted with one or more groups selected from halo, C1-C4 alkyl, C1-C4 heteroalkyl, C1-C6 acyl, C1-C6 heteroacyl, hydroxy, amino, and =O;
and wherein two R' on the same or adjacent atoms can be linked to form a 3-7 membered ring optionally containing up to three heteroatoms selected from N, O and S; and
p is 0-2;
or a pharmaceutically acceptable salt thereof.

2. A compound according to claim 1, wherein Q¹ is S.

3. A compound according to claim 1, wherein Q² is S.

4. A compound according to claim 1, wherein Q³ is S.

5. A compound according to any of claims 1-4, wherein Z is a bond and R³ is optionally substituted aryl.

6. A compound of any according to any of claims 1-5, wherein Het represents an optionally substituted pyrimidine ring.

7. A compound according to any of claims 1-5, wherein Het represents an optionally substituted triazine ring.

8. A compound according to claims 6 or 7, wherein L is CHR².

9. A compound according to claims 6 or 7, wherein L is -CHR²-NR²-.

10. A compound according to claims 6 or 7, wherein L is -CHR²-S(O)ₙ-, and n is 0 or 2.

11. A compound according to any of claims 8-14, wherein L contains a chiral center that is in the S stereochemical configuration.

12. A compound according to any preceding claim for use in the treatment of any of: a hematologic cancer, an inflammatory disorder, an immune disorder, and hypertension.

13. A compound for use according to claim 12, wherein the hematologic cancer is selected from acute lymphoblastic leukemia, acute myeloid leukemia, chronic lymphocytic leukemia, chronic myelogenous leukemia, and hairy cell leukemia, and
wherein the inflammatory disorder or immune disorder is selected from rheumatoid arthritis, multiple sclerosis, asthma, systemic lupus erythematosus, scleroderma, Sjögren's syndrome, myasthenia gravis, Guillain-Barré syndrome, Hashimoto's thyroiditis, Graves' disease, inflammatory bowel disease (IBD), Crohn's disease, ulcerative colitis, vasculitis, hemolytic anemia, thrombocytopenia, psoriasis, type I (insulin dependent) diabetes, and allergic rhinitis.

14. A pharmaceutical composition comprising a compound according to any of claims 1-11, admixed with at least one pharmaceutically acceptable excipient.

15. Use of a compound of any according to claims 1-11 in the manufacture of a medicament.

16. Use according to claim 15, wherein the medicament is a medicament for the treatment of a hematological cancer, an inflammatory disorder, an immune disorder, or hypertension.

17. Use according to claim 16, wherein the hematologic cancer is selected from acute lymphoblastic leukemia, acute myeloid leukemia, chronic lymphocytic leukemia, chronic myelogenous leukemia, and hairy cell leukemia, and
wherein the inflammatory disorder or immune disorder is selected from rheumatoid arthritis, multiple sclerosis, asthma, systemic lupus erythematosus, scleroderma, Sjögren's syndrome, myasthenia gravis, Guillain-Barré syndrome, Hashimoto's thyroiditis, Graves' disease, inflammatory bowel disease (IBD), Crohn's disease, ulcerative colitis, vasculitis, hemolytic anemia, thrombocytopenia, psoriasis, type I (insulin dependent) diabetes, and allergic rhinitis.
